(19) Europäisches Patentamt
European Patent Office
Office européen des brevets

(11) Publication number: **0 034 349**
**B1**

(12) # EUROPEAN PATENT SPECIFICATION

(45) Date of publication of patent specification: **14.12.83**

(21) Application number: **81101025.5**

(22) Date of filing: **13.02.81**

(51) Int. Cl.³: **A 61 K 31/395,**
C 07 D 401/12
//C07D213/79, C07D213/84,
C07D213/26, C07D213/89,
C07D309/38, C07D213/81

(54) **Novel pyridinecarboxamide derivatives, a process for the preparation thereof, the use of said derivatives and a pharmaceutical composition containing same.**

(30) Priority: **15.02.80 JP 18008/80**

(43) Date of publication of application:
**26.08.81 Bulletin 81/34**

(45) Publication of the grant of the patent:
**14.12.83 Bulletin 83/50**

(84) Designated Contracting States:
**CH DE FR GB IT LI**

(56) References cited:
**DE - A - 2 749 518**
**US - A - 3 197 473**
**US - A - 4 123 432**

**Chemical abstracts, vol. 95, 1981 page 764, abstract 43129c Columbus, Ohio, US**

(73) Proprietor: **Tanabe Seiyaku Co., Ltd.**
**No. 21 Dosho-machi 3-chome Higashi-ku**
**Osaka-shi Osaka-fu (JP)**

(72) Inventor: **Honma, Yasushi Nishi-Ageo-Daiichi-Danchi 3-20-501**
**No. 845-1, Oaza-Koshikiya**
**Ageo-shi Saitama-ken (JP)**
Inventor: **Takeda, Mikio**
**No. 323-70, Tsukamoto**
**Urawa-shi Saitama-ken (JP)**
Inventor: **Tsuzurahara, Kei**
**No. 13-15, Fujimi 2-chome**
**Ageo-shi Saitama-ken (JP)**

(74) Representative: **Hansen, Bernd, Dr.rer.nat. et al,**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 349 B1

# Novel pyridinecarboxamide derivatives, a process for the preparation thereof, the use of said derivatives and a pharmaceutical composition containing them

The present invention relates to novel pyridinecarboxamide derivatives, a process for the preparation thereof, the use of said derivatives and a pharmaceutical composition containing same. More particularly, it relates to N-(5-tetrazolyl)-6-phenyl-2-pyridinecarboxamide derivatives of the formula:

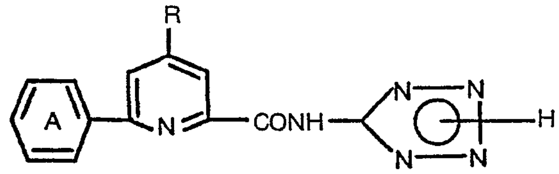

[I]

wherein R is hydrogen, a halogen, a alkoxy ($C_{1-3}$) or a alkyl ($C_{1-4}$), and the phenyl ring A is a phenyl having a substituent selected from amino, a monoalkyl($C_{1-2}$)amino, a dialkyl($C_{1-2}$)amino, or acetylamino, or a pharmaceutically acceptable salt thereof, and a process for the preparation thereof.

The compounds [I] of the present invention have two isomeric structures: 1H-isomer and 2H-isomer in the tetrazole ring as shown in the following formulae, which are mutually converted from one to another. These isomers are both included within the present invention.

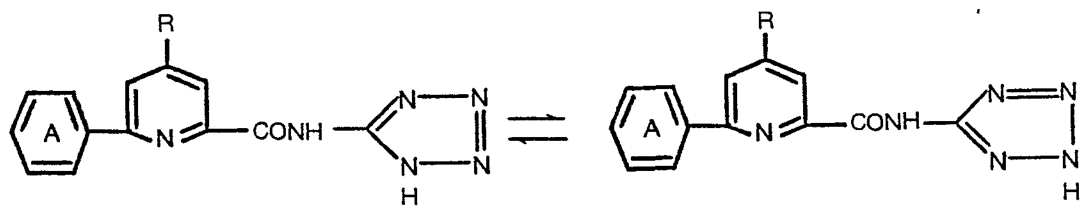

wherein R and the ring A are as defined above.

The compounds [I] of the present invention are novel compounds and have excellent anti-allergic activities and are useful as a medicine. Particularly, the compounds [I] are characteristic in that they can show excellent anti-allergic activities even by administration orally.

In the formula [I], the group "R" include hydrogen, a halogen, an alkyl having 1 to 4 carbon atoms, and an alkoxy having 1 to 3 carbon atoms, and preferable R groups are hydrogen, chlorine, methyl, ethyl, propyl, isopropyl, butyl, methoxy, ethoxy, propoxy, or isopropoxy.

The phenyl ring A has a substituent selected from amino, an amino substituted with one or two alkyl groups having 1 to 2 carbon atoms, and acetylamino. Suitable examples of the phenyl ring A are an aminophenyl (e.g. 3-aminophenyl, 4-aminophenyl), a monoalkyl($C_{1-2}$)aminophenyl (e.g. 3-methylaminophenyl, 4-methylaminophenyl, 3-ethylaminophenyl, 4-ethylaminophenyl), a dialkyl($C_{1-2}$)aminophenyl (e.g. 3-dimethylaminophenyl, 4-dimethylaminophenyl, 3-diethylaminophenyl, 4-diethylaminophenyl), and an acetylaminophenyl (e.g. 3-acetamidophenyl, 4-acetamidophenyl). Among the compounds (I) of the present invention, the most preferable compound is N-(5-tetrazolyl)-4-methyl-6-(4-methylaminophenyl)-2-pyridinecarboxamide.

The compounds [I] can be used as a medicine in the form of a free compound or a pharmaceutically acceptable salt thereof. Suitable examples of the pharmaceutically acceptable salt are an alkali metal salt (e.g. sodium salt, potassium salt, lithium salt), an organic amine salt (e.g. triethanolamine salt, tris(hydroxymethyl)aminomethane salt), a basic amino acid salt (e.g. lysine salt), an inorganic acid addition salt (e.g. hydrochloride, sulfate, phosphate), nitrate or an organic acid addition salt (e.g. acetate, lactate, tartrate, fumarate, maleate, oxalate, succinate, methanesulfonate, benzoate).

The compounds [I] or their salts can be administered orally or parenterally when used as a medicine. These compounds may be used in admixture with conventional pharmaceutically acceptable carrier or diluent. Suitable examples of the carrier or diluent are gum arabic, gelatine, sorbitol, tragacanth gum, polyvinylpyrrolidone, lactose, sucrose, potassium phosphate, magnesium stearate, talc or potato starch.

The compounds of the present invention and compositions thereof can be used in conventional types of preparations, for example, solid preparations such as tablets, pills, powders, capsules, granules, or liquid preparations such as emulsions, suspensions or solutions. When they are administered parenterally, they may be used in the form of an injection.

The therapeutic dose of the pyridinecarboxamide derivative [I] or its salt depends on route of administration; the conditions of diseases; and the particular diseases to be treated. In general, it may be used at a dose of about 0.2 to about 120 mg (in terms of free base), especially about 1 to about 60 mg (in terms of free base), per kilogram of body weight per day in the case of oral administration. Further, it may be used at a dose of about 0.001 to about 20 mg (in terms of free base), especially

about 0.003 to about 3 mg (in terms of free base), per kilogram of body weight per day in the case of intravenous administration.

The compounds [I] of the present invention (i.e. the compounds of the formulae [I—A], [I—B] and [I—C]) can easily be prepared by the reaction as shown in the following reaction schemes:

Reaction Scheme—I

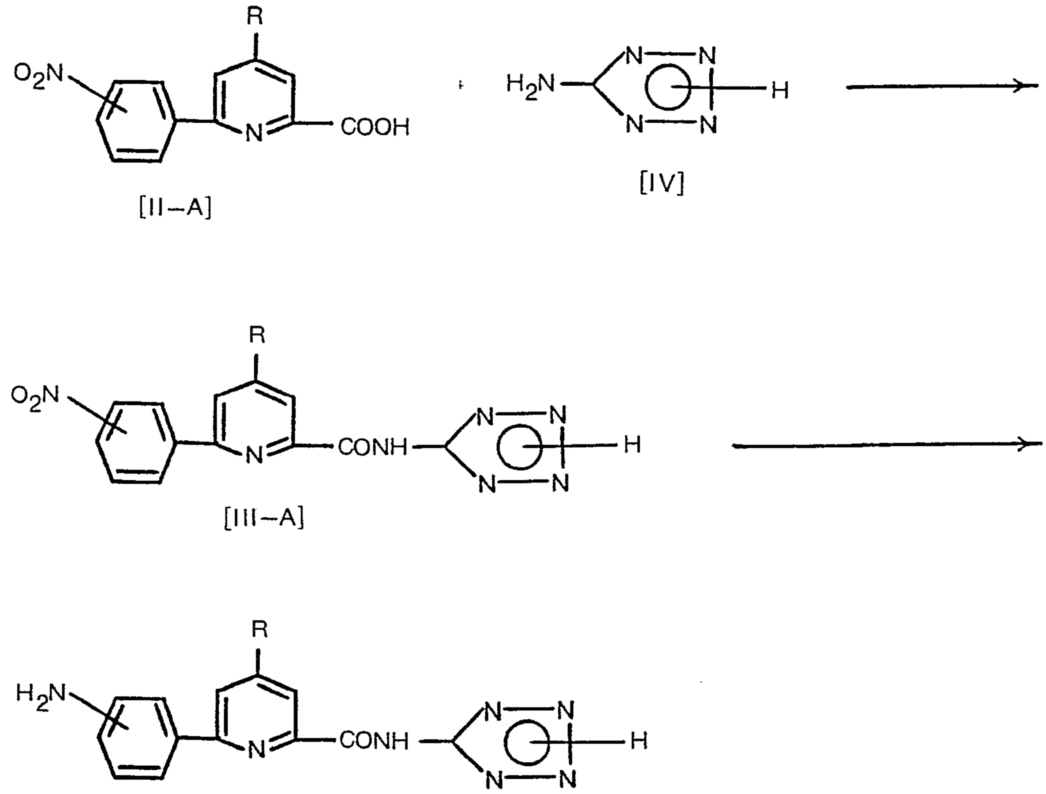

[II—A] [IV]

[III—A]

[I—A]

Reaction Scheme—II:

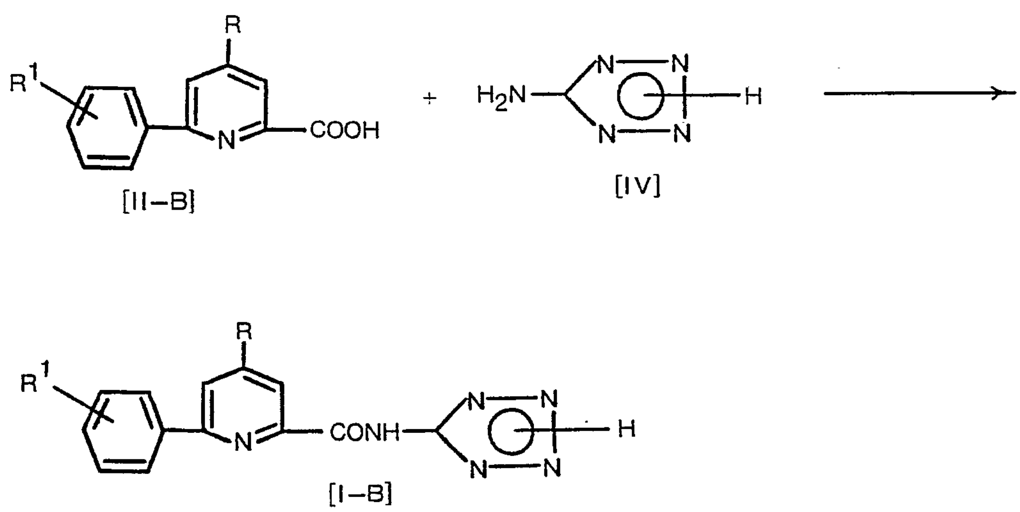

[II—B] [IV]

[I—B]

Reaction Scheme—III

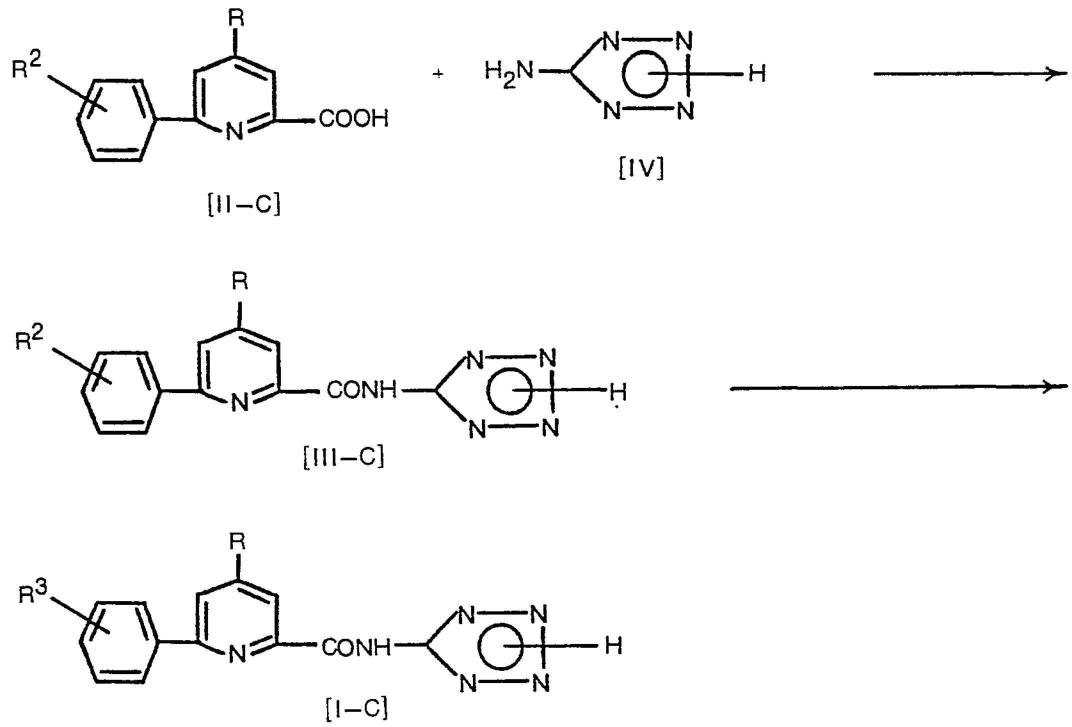

[II—C] [IV] [III—C] [I—C]

In the above formulae, R is as defined above, $R^1$ is a dialkyl($C_{1-2}$)amino or a acetylamino which are the same as a part of the substituents on the phenyl ring A as defined above, $R^2$ is amino or a monoalkyl($C_{1-2}$)amino which are the same as a part of the substituents on the phenyl ring A as defined above, provided that said amino group is protected by an appropriate protecting group such as benzyloxycarbonyl, p-methoxybenzyloxycarbonyl, tert-butoxycarbonyl, tert-amyloxycarbonyl, and $R^3$ is a group obtained after removing the amino-protecting group from the above groups for $R^2$.

That is, the compounds [I—A] can be prepared by subjecting 6-phenyl-2-pyridinecarboxylic acid derivative [II—A] and 5-aminotetrazole [IV] to condensation reaction, and reducing the resulting compound [III—A] in order to convert the nitro group into amino group. The compound [I—B] can be prepared by subjecting 6-phenyl-2-pyridinecarboxylic acid derivative [II—B] and 5-aminotetrazole [IV] to condensation reaction. The compounds [I—C] can be prepared by subjecting 6-phenyl-2-pyridinecarboxylic acid derivative [II—C] and 5-aminotetrazole [IV] to condensation reaction, and removing the amino-protecting group of the resulting compound [III—C].

These reactions are explained in more detail below.

Condensation reaction:

The condensation reaction of the starting compounds [II—A], [II—B] and [II—C] with 5-aminotetrazole [IV] can be carried out by conventional methods which are usually used for forming an acid amide bond in peptide chemistry. For instance, when a free carboxylic acid compound is used as the starting material, the reaction can be carried out in an appropriate solvent in the presence of an appropriate condensing agent. Suitable examples of the condensing agent are N,N'-carbonyldiimidazole, N,N'-dicyclohexylcarbodiimide. Suitable examples of the solvent are tetrahydrofuran, dioxane, dimethylformamide. The reaction proceeds preferably at a temperature of −10° to 100°C.

When a reactive derivative of carboxylic acid is used as the starting material, the reaction can be carried out by the conventional acid halide method, mixed acid anhydride method, etc., preferably acid halide method. The acid halide method can be carried out by reacting with an acid halide of the compound [II—A], [II—B] or [II—C] with 5-aminotetrazole in an appropriate solvent in the presence of an appropriate acid acceptor. Suitable examples of the acid acceptor are organic bases (e.g. triethylamine, pyridine), alkali metal carbonates (e.g. sodium carbonate, sodium hydrogen carbonate). Suitable examples of the solvent are dimethylformamide, dioxane, etc. When an organic base is used in a large amount as the acid acceptor, it acts also as a solvent, and hence no further solvent is required. The reaction proceeds preferably at a temperature of 20° to 120°C.

The acid halide (e.g. acid chloride) to the compound [II—A], [II—B] or [II—C] can be obtained by treating the corresponding free carboxylic acid with a halogenating agent, such as thionyl chloride, phosphorus oxychloride, phosphorus pentachloride. The halogenating reaction is preferably carried out in an appropriate solvent at a temperature of from 0°C to the reflux temperature of the halogenating

agent. Suitable examples of the solvent are benzene, toluene, pyridine, etc., but when the halogenating agent is used in an excess amount, the halogenating agent acts also as the solvent and hence further solvent is not necessarily required.

When the condensation reaction is carried out by a mixed acid anhydride method, the starting compound [II—A], [II—B] or [II—C] is reacted with ethyl chloroformate, isobutyl chloroformate, or the like, and the resulting mixed acid anhydride of the compound [II—A], [II—B] or [II—C] is reacted with 5-aminotetrazole in an appropriate solvent (e.g. tetrahydrofuran, dioxane, dimethylformamide, or a mixture thereof) at a temperature of −30° to 20°C.

Reduction reaction:

The reduction of the compounds [III—A] thus obtained is carried out by all conventional methods which are usually used for reducing nitro group to amino group, for example a catalytic reduction method, a method using an acid (e.g. acetic acid, hydrochloric acid) and a metal (e.g. zinc, tin). The catalytic reduction method is usually carried out in hydrogen gas in the presence of a catalyst such as palladium catalysts (e.g. palladium black, palladium carbon), or Raney nickel catalyst. The catalytic reduction is preferably carried out in the presence of an appropriate alkali, or after converting the compounds [III—A] into an alkaline salt thereof, because the product can be obtained in a higher yield. Suitable examples of the alkali are alkali metal hydroxides (e.g. potassium hydroxide, sodium hydroxide), alkali metal carbonates (e.g. potassium carbonate, sodium carbonate). The reaction is carried out in an appropriate inert solvent, such as a lower alkanol (e.g. methanol, ethanol), dimethylformamide, tetrahydrofuran, water, or the like.

When the reduction is carried out by using an acid and a metal, it is done by treating the compound to be reduced with a metal such as iron, tin or zinc in hydrochloric acid or acetic acid at a temperature of 0° to 150°C, preferably 50° to 80°C.

Removal of the amino-protecting group:

The removal of the amino-protecting group from the compounds [III—C] can be carried out by various methods in accordance with the kinds of the protecting groups. For instance, when benzyloxycarbonyl or p-methoxybenzyloxycarbonyl group is used as the amino-protecting group, the removal reaction can preferably be carried out by catalytic reduction method or treatment with an acid. The removal of the amino-protecting group by catalytic reduction is carried out in hydrogen gas stream in the presence of a catalyst such as palladium black, palladium carbon, palladium oxide, colloidal palladium or platinum. This reaction is preferably carried out in the presence of an appropriate alkali, or after converting the compound [III—C] into an alkaline salt thereof, because the product can be obtained in a higher yield. The reaction is carried out in an appropriate inert solvent, such as a lower alkanol (e.g. methanol, ethanol), dimethylformamide, tetrahydrofuran, or water at room temperature or an elevated temperature and under atmospheric pressure or under increased pressure (e.g. about 30° to 100°C, about 490 to 4,900 kPa). The reduction can also be carried out by treatment with an acid, for example by treating the compound with hydrobromic acid in acetic acid, or by treating the compound with trifluoroacetic acid in methanol, at a temperature of about −10° to 50°C, preferably at about 0° to 20°C.

When tert-butoxycarbonyl or tert-amyloxycarbonyl group is used as the amino-protecting group, the removal of the amino-protecting group is preferably carried out by treating the compound with hydrochloric acid or hydrobromic acid in acetic acid, or by treating the compound with trifluoroacetic acid in methanol, at a temperature of about −10° to 50°C, particularly about 0° to 20°C.

Besides, when the compounds [III—C] has a halogen substituent at 4-position of pyridine nucleus, the halogen substituent is also removed by the catalytic reduction, and hence, in case of 4-halogenated compounds, the removal of the amino-protecting group is preferably carried out by the treatment with an acid.

The compounds [I] of the present invention can be converted into their pharmaceutically acceptable salt in usual manner. For instance, when the free compounds [I] are treated with an alkali metal hydroxide (e.g. sodium hydroxide, potassium hydroxide, lithium hydroxide), an alkali metal carbonate (e.g. sodium carbonate, potassium carbonate, sodium hydrogen carbonate), an organic amine (e.g. triethanolamine, tris(hydroxymethyl)aminomethane), or a basic amino acid (e.g. lysine), they are converted into an alkaline salt of the tetrazole moiety thereof. Besides, when the compounds are treated with an inorganic acid (e.g. hydrochloric acid, sulfuric acid, phosphoric acid, nitric acid), or an organic acid (e.g. acetic acid, lactic acid, tartaric acid, oxalic acid), they are converted into an acid addition salt of the amino moiety on the phenyl ring thereof.

The starting compounds [II], (i.e. the compounds of the formulae [II—A], [II—B], and [II—C]) may be prepared, for example, by the following processes.

(a) The starting compounds [II—A] wherein R is hydrogen or a lower alkyl can be prepared by treating a 2-phenylpyridine compound of the formula:

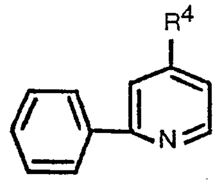

(1)

wherein $R^4$ is hydrogen or an alkyl($C_{1-4}$), with nitric acid and sulfuric acid, by which a nitro group is introduced onto the phenyl ring of the compound [1], treating the resulting nitro-substituted phenyl derivative with hydrogen peroxide to obtain the corresponding N-oxide compound, reacting the resulting N-oxide compound with dimethyl sulfate to give the corresponding N-methoxypyridinium compound of the formula:

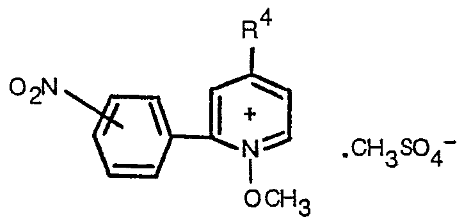

(2)

wherein $R^4$ is as defined above, reacting the compound with an alkali metal cyanide to introduce cyano group at 2-position of the pyridine ring, and then hydrolyzing the resulting 2-cyano compound with hydrochloric acid.

(b) The starting compounds [II—A] wherein R is an alkoxy($C_{1-3}$) can be prepared by reacting nitrobenzalacetone and diethyl oxalate in the presence of an alkali metal hydride to give ethyl 6-(nitrophenyl)-2,4-dioxohex-5-enoate, treating the resulting compound with bromine and then with potassium acetate to give a compound of the formula:

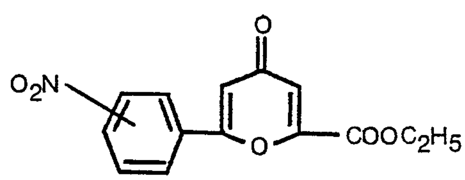

(3)

reacting the compound with ammonia in ethanol, hydrolyzing the resulting compound with hydrochloric acid to give a compound of the formula:

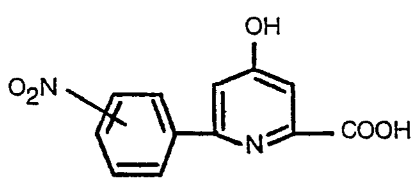

(4)

reacting the compound with a $C_{1-3}$ alkyl iodide, by which 4-hydroxy group on the pyridine ring and the carboxy group are alkylated, and then hydrolyzing the resulting alkylated compound with an alkali.

(c) The starting compounds [II—A] wherein R is a halogen atom can be prepared by reacting a compound of the above formula (4) with a lower alkanol in the presence of a thionyl halide to give the corresponding lower alkyl ester of the formula:

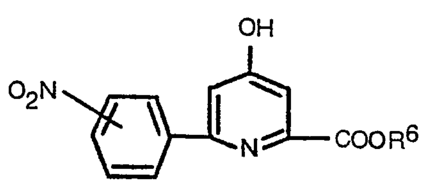

(5)

wherein $R^6$ is a lower alkyl, reacting the compound with a halogenating agent (e.g. thionyl halide), by which 4-hydroxy group on the pyridine ring of the ester compound (5) is substituted with a halogen atom, and hydrolyzing the resulting. compound with an alkali.

(d) The starting compounds [II—B] wherein R is hydrogen or a ($C_{1-4}$) alkyl and $R^1$ is a dialkyl[$C_{1-2}$)amino can be prepared by subjecting the compound [II—A] obtained in the above (a) wherein R is hydrogen or a ($C_{1-4}$) alkyl to catalytic reduction, whereby the nitro group is converted into amino group, reacting the resulting amino compound with a lower alkanol in the presence of thionyl chloride to give the corresponding lower alkyl ester of the formula:

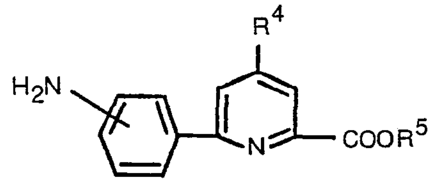

(6)

wherein $R^4$ is as defined above, and $R^5$ is a lower alkyl, reacting the compound with a $C_{1-2}$ alkyl iodide in the presence of an anhydrous alkali metal carbonate to give the corresponding dialkyl($C_{1-2}$)amino compound, and then hydrolyzing the resulting compound with an alkali.

(e) The starting compounds [II—B] wherein R is hydrogen or a ($C_{1-4}$) alkyl and $R^1$ is a acetylamino can be prepared by reacting a compound of the formula (6) with acetic anhydride under heating in the presence of pyridine.

(f) The starting compounds [II—B] wherein R is a ($C_{1-3}$) alkoxy and $R^1$ is a monoalkyl($C_{1-2}$)amino or a dialkyl($C_{1-2}$)amino can be prepared by subjecting a compound of the above formula (5) to catalytic reduction, whereby the nitro group is converted into amino group, to give a compound of the formula:

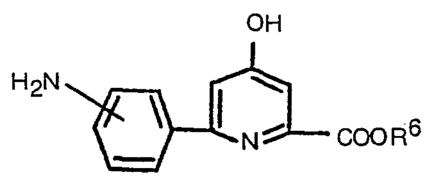

(7)

wherein $R^6$ is a lower alkyl, reacting the compound with a ($C_{1-3}$) alkyl iodide in the presence of an anhydrous alkali metal carbonate as in the above (d), and then hydrolyzing the resulting compound with an alkali.

(g) The starting compounds [II—B] wherein R is a ($C_{1-3}$) alkoxy and $R^1$ is a acetylamino can be prepared by reacting a compound of the above formula (7) with acetic anhydride as in the above (e) to give the corresponding N,O-diacetyl compound, hydrolyzing the compound with an alkali to give the corresponding monoacetyl compound of the formula:

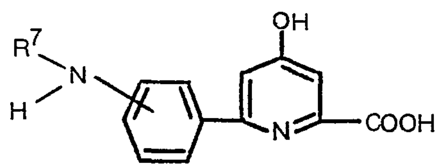

(8)

wherein $R^7$ is an acetyl group, subjecting the compound to O-alkylation as in the above (f), and hydrolyzing the resulting compound with an alkali.

(h) The starting compounds [II—B] wherein $R^1$ is a acetylamino and R is a halogen atom can be prepared by converting the 4-hydroxy group on the pyridine ring of a compound of the above formula (8) into a halogen atom in the same manner as described in the above (c), and then hydrolyzing the resulting compound with an alkali,

(i) The starting compounds [II—C] wherein R is hydrogen or a ($C_{1-4}$) alkyl and $R^2$ is monoalkyl($C_{1-2}$)amino having a protecting group on the amino group can be prepared by reacting a compound of the above formula (6) with an amino-protecting group introducing agent (e.g. benzyloxycarbonyl chloride) in a conventional manner, reacting the resulting compound with a ($C_{1-4}$) alkyl iodide in the same manner as described in the above (d), and then hydrolyzing the resulting compound with an alkali.

(j) The starting compounds [II—C] wherein R is a ($C_{1-3}$) alkoxy and $R^2$ is a protected amino group can be prepared by introducing a protecting group to the amino group of a compound of the above formula (7) in the same manner as described in the above (i) to give a compound of the formula:

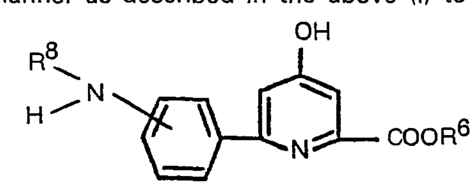

(9)

wherein $R^8$ is an amino-protecting group and $R^6$ is as defined above, subjecting the compound to O-alkylation of 4-hydroxy group of the pyridine ring thereof in the same manner as described in the above (f), and then hydrolyzing the resulting compound with an alkali.

(k) The starting compounds [II—C] wherein R is a $(C_{1-3})$ alkoxy and $R^2$ is a monoalkyl$(C_{1-2})$amino having a protecting group on the amino group can be prepared by subjecting a compound of the above formula (9) to O-alkylation of 4-hydroxy group on the pyridine ring thereof in the same manner as described in the above (f), reacting the resulting O-lower alkylated compound with a $(C_{1-2})$ alkyl iodide in the presence of an anhydrous alkali metal carbonate as in the above (d), and then hydrolyzing the resulting compound with an alkali.

(l) The starting compounds [II—C] wherein R is a hydrogen atom and $R^2$ is protected amino can be prepared by halogenating the 4-hydroxy group on the pyridine ring of a compound of the above formula (9) in the same manner as described in the above (h), and then hydrolyzing the resulting compound with an alkali.

## *Experiment*

The anti-allergic activities of the present compounds were tested by passive cutaneous anaphylaxis (PCA) reaction.

Method:

Male Sprague Dawley rats weighing about 200 g (3—4 rats per group) were used. The skin of their backs was shaved. Antiserum *1 (0.05 ml) to Ascaris suum, diluted 40-fold, was subcutaneously administered to rats. After about 24 hours, a mixture (1 ml) of Ascaris suum extract*2 (0.5 mg protein) and Evans blue (5 mg) was injected intravenously in tail, and after 30 minutes, the size (long length × short length) of the blue spots appeared on the skin was measured.

The test compound was dissolved or suspended in a saline solution containing 0.5% carboxymethylcellulose and was orally administered 15 minutes before the administration of the mixture of Ascaris suum extract and Evans blue.

The PCA inhibitory activity was shown by the amount ($ID_{40}$) of the test compound which was necessary to make 40% smaller the size of the blue spots compared to that in the animals to which no test compound was administered:

---

[Remarks]:

*1) Antiserum to Ascaris suum was prepared as follows:

Male Sprague Dawley rats, weighing about 200 mg, were immunized with Ascaris suum extract*2 (2 mg protein/0.25 ml) which was administered subcutaneously in the inguinal region, and Bordetella pertussis ($2 \times 10^{10}$/0.5 ml) was administered intraperitoneally (primary immunization). One week later, Ascaris suum extract (0.2 mg protein/0.25 ml) was again administered subcutaneously in the inguinal region (secondary immunization). After three weeks, the blood was taken out and the serum was preserved frozen. The antiserum thus obtained was rich in IgE antibody in view of the sensivity to heat.

*2) Ascaris suum extract (antigen) was prepared as follows:

Ascaris suum (20 g) was homogenized in physiological saline (135 ml) with Polytron®, and the mixture was centrifuged. The supernatant fluid was dialyzed with borate (12.5 mM)-buffered saline (pH 8.0), and the resulting liquid was used as Ascaris suum extract (preserved frozen). As a result of quantitative analysis according to the Lowry's method, it contained 5.7 mg protein/ml.

Results:

The test compounds shown in Table 1 were administered orally, and the anti-allergic activities were measured. The results are shown in Table 1. Disodium cromoglycate (Intal®), a commercially available anti-allergic drug, when orally administered (20 mg/kg), showed no activity.

TABLE 1

| 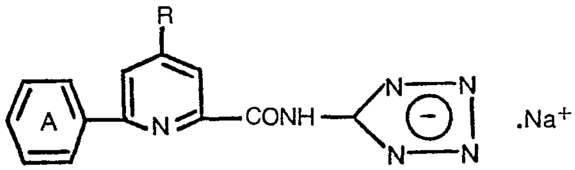 | | | PCA inhibitory activity ($ID_{40}$, mg/kg) |
|---|---|---|---|
| No. | Ring A | R | |
| 1 | 4-Dimethylaminophenyl | Methoxy | 0.5 |
| 2 | 4-Dimethylaminophenyl | Methyl | 0.3 |
| 3 | 4-Diethylaminophenyl | Methyl | 0.3 |
| 4 | 4-Acetylaminophenyl | Methoxy | 3.4 |
| 5 | 4-Diethylaminophenyl | Ethoxy | 0.2 |
| 6 | 4-Ethylaminophenyl | Ethoxy | 0.1 |
| 7 | 4-Acetylaminophenyl | Methyl | 3.0 |
| 8 | 4-Methylaminophenyl | Hydrogen | 0.4 |
| 9 | 4-Methylaminophenyl | Methyl | 0.4 |
| 10 | 4-Methylaminophenyl | Methoxy | 1.5 |
| 11 | 4-Aminophenyl | Ethoxy | 0.4 |
| 12 | 4-Aminophenyl | n-Propoxy | 0.4 |
| 13 | 4-Aminophenyl | Hydrogen | 0.7 |
| 14 | 4-Aminophenyl | Methyl | 0.2 |
| 15 | 3-Aminophenyl | Methyl | 1.3 |
| 16 | 4-Aminophenyl | Isopropyl | 3.0 |
| 17 | 4-Aminophenyl | n-Butyl | 0.3 |
| 18 | 4-Aminophenyl | Methoxy | 0.6 |
| 19 | 4-Aminophenyl | Chlorine | 0.3 |

The present invention is illustrated by the following Examples but is not construed as limiting thereto.

Example 1

(1) To a solution of 4-methoxy-6-(4-dimethylaminophenyl)-2-pyridinecarboxylic acid (0.44 g) in dimethylformamide (6 ml) is added carbonyldiimidazole (0.29 g), and the mixture is agitated at room temperature for one hour. To the mixture is added 5-aminotetrazole (0.15 g), and the mixture is heated at 70°C for one hour. After cooling, the reaction mixture is poured into ice-water. The resulting precipitates are collected by filtration and washed with water and ethanol to give N-(5-tetrazolyl)-4-methoxy-6-(4-dimethylaminophenyl)-2-pyridinecarboxamide (0.36 g). M.P. 261°—263°C (recrystallized from dimethylformamide-ethanol).

(2) A mixture of N-(5-tetrazolyl)-4-methoxy-6-(4-dimethylaminophenyl)-2-pyridinecarboxamide (0.34 g) obtained in the above (1), 1N sodium hydroxide (1 ml), dimethylformamide (3 ml) and ethanol (10 ml) are dissolved with heating and concentrated under reduced pressure. To the resulting residue is added isopropanol, and the precipitated crystals are collected by filtration and dried to give N-(5-tetrazolyl)-4-methoxy-6-(4-dimethylaminophenyl)-2-pyridinecarboxamide sodium salt (0.33 g). M.P. 240°—245°C (decomp.) (wetted from 230°C).

Example 2

(1) In the same manner as described in Example 1 — (1), 4-methyl-6-(4-dimethylaminophenyl)-2-pyridinecarboxylic acid (1.25 g), carbonyldiimidazole (1.38 g) and 5-aminotetrazole (0.47 g) are reacted to give N-(5-tetrazolyl)-4-methyl-6-(4-dimethylaminophenyl)-2-pyridinecarboxamide (0.3 g). M.P. 265°—269°C (decomp.) (wetted from 255°C) (recrystallized from dimethylformamide-ethanol).

(2) N-(5-Tetrazolyl)-4-methyl-6-(4-dimethylaminophenyl)-2-pyridinecarboxamide obtained in (1) is treated in the same manner as described in Example 1 — (2) to give N-(5-tetrazolyl)-4-methyl-6-(4-dimethylaminophenyl)-2-pyridinecarboxamide sodium salt. M.P. 238°—248°C (decomp.) (wetted from 220°C).

Example 3

(1) In the same manner as described in Example 1 — (1), 4-methyl-6-(4-dimethylaminophenyl)-2-pyridinecarboxylic acid (1.0 g), carbonyldiimidazole (1.0 g) and 5-aminotetrazole (0.32 g) are reacted to give N-(5-tetrazolyl)-4-methyl-6-(4-diethylaminophenyl)-2-pyridinecarboxamide (0.81 g). M.P. 254°—256°C (decomp.) (recrytallized from dimethylformamide-ethanol).

(2) N-(5-Tetrazolyl)-4-methyl-6-(4-diethylaminophenyl)-2-pyridinecarboxamide obtained in (1) is treated in the same manner as described in Example 1 — (2) to give N-(5-tetrazolyl)-4-methyl-6-(4-dimethylaminophenyl)-2-pyridinecarboxamide sodium salt. M.P. 290°—295°C (decomp.) (wetted from 225°C).

Example 4

(1) In the same manner as described in Example 1 — (1), 4-methoxy-6-(4-acetylaminophenyl)-2-pyridinecarboxylic acid (0.46 g), carbonyldiimidazole (0.29 g) and 5-aminotetrazole (0.15 g) are reacted to give N-(5-tetrazolyl)-4-methoxy-6-(4-acetylaminophenyl)-2-pyridinecarboxamide (0.48 g). M.P. 286°—288°C (decomp.) (recrystallized from dimethylformamide-ethanol).

(2) N-(5-Tetrazolyl-4-methoxy-6-(4-acetylaminophenyl)-2-pyridinecarboxamide obtained in (1) is treated in the same manner as described in Example 1 — (2) to give N-(5-tetrazolyl)-4-methoxy-6-(4-acetylaminophenyl)-2-pyridinecarboxamide sodium salt. M.P. 275°—280°C (decomp.)

Example 5

(1) In the same manner as described in Example 1 — (1), 4-ethoxy-6-(4-diethylaminophenyl)-2-pyridinecarboxylic acid (0.56 g), carbonyldiimidazole (0.32 g) and 5-aminotetrazole (0.17 g) are reacted to give N-(5-tetrazolyl)-4-ethoxy-6-(4-diethylaminophenyl)-2-pyridinecarboxamide (0.52 g). M.P. 228°—230°C (decomp.) (recrystallized from dimethylformamide-ethanol).

(2) N-(5-Tetrazolyl)-4-ethoxy-6-(4-diethylaminophenyl)-2-pyridinecarboxamide obtained in (1) is treated in the same manner as described in Example 1 — (2) to give N-(5-tetrazolyl)-4-ethoxy-6-(4-diethylaminophenyl)-2-pyridinecarboxamide sodium salt. M.P. decomposed gradually from 210°C (wetted from 170°C).

Example 6

(1) In the same manner as described in Example 1 — (1), 4-ethoxy-6-(4-ethylaminophenyl)-2-pyridinecarboxylic acid (0.28 g), carbonyldiimidazole (0.17 g) and 5-aminotetrazole (0.09 g) are reacted to give N-(5-tetrazolyl)-4-ethoxy-6-(4-ethylaminophenyl)-2-pyridinecarboxamide (0.24 g). M.P. 255°—257°C (decomp.) (recrystallized from dimethylformamide-ethanol).

(2) N-(5-Tetrazolyl)-4-ethoxy-6-(4-ethylaminophenyl)-2-pyridinecarboxamide obtained in (1) is treated in the same manner as described in Example 1 — (2) to give N-(5-tetrazolyl)-4-ethoxy-6-(4-ethylaminophenyl)-2-pyridinecarboxamide sodium salt. M.P. decomposed gradually from 190°C (wetted at 90°C).

Example 7

(1) In the same manner as described in Example 1 — (1), 4-methyl-6-(4-acetylaminophenyl)-2-pyridinecarboxylic acid (1.0 g), carbonyldiimidazole (0.72 g) and 5-aminotetrazole (0.38 g) are reacted to give N-(5-tetrazolyl)-4-methyl-6-(4-acetylaminophenyl)-2-pyridinecarboxamide (0.9 g). M.P. 309°—310°C (decomp.) (wetted at about 290°C) (recrystallized from dimethylformamide-ethanol).

(2) N-(5-Tetrazolyl)-4-methyl-6-(4-acetylaminophenyl)-2-pyridinecarboxamide obtained in (1) is treated in the same manner as described in Example 1 — (2) to give N-(5-tetrazolyl)-4-methyl-6-(4-acetylaminophenyl)-2-pyridinecarboxamide sodium salt. M.P. above 300°C.

Example 8

(1) In the same manner as described in Example 1 — (1), 6-(4-N-methyl-N-benzyloxycarbonylaminophenyl)-2-pyridinecarboxylic acid (3.39 g), carbonyldiimidazole (1.69 g) and 5-aminotetrazole (0.85 g) are reacted to give N-(5-tetrazolyl)-6-(4-N-methyl-N-benzyloxycarbonylaminophenyl)-2-pyridinecarboxamide (2.2 g). M.P. 249°C (decomp.) (wetted at about 240°C).

(2) A mixture of N-(5-tetrazolyl)-6-(4-N-methyl-N-benzyloxycarbonylaminophenyl)-2-pyridinecarboxamide (2.2 g) obtained in (1), 10% aqueous sodium hydroxide (5 ml), water (150 ml), ethanol (100 ml) and 10% palladium-carbon (1 g) is shaken under hydrogen gas at room temperature for 2 hours. After removing the catalyst by filtration, the resulting filtrate is made acidic with diluted hydrochloric acid, and then the mixture is concentrated under reduced pressure to remove the solvent. To the resulting residue is added ethanol, and precipitated crystals are collected by filtration. The crystals are recrystallized from dimethylformamide-ethanol to give N-(5-tetrazolyl)-6-(4-methylaminophenyl)-2-pyridinecarboxamide (1.35 g). M.P. 264°—266°C (decomp.) (recrystallized from dimethylformamide-ethanol).

(3) N-(5-Tetrazolyl)-6-(4-methylaminophenyl)-2-pyridinecarboxamide obtained in (2) is treated in the same manner as described in Example 1 — (2) to give N-(5-tetrazolyl)-6-(4-methylaminophenyl)-2-pyridinecarboxamide sodium salt. M.P. above 300°C.

Example 9

(1) In the same manner as described in Example 1 — (1), 4-methyl-6-(4-N-methyl-N-benzyloxycarbonylaminophenyl)-2-pyridinecarboxylic acid (1.0 g), carbonyldiimidazole (0.47 g) and 5-aminotetrazole (0.27 g) are reacted to give N-(5-tetrazolyl)-4-methyl-6-(4-N-methyl-N-benzyloxycarbonylaminophenyl)-2-pyridinecarboxamide (0.49 g). M.P. 220°—221°C (decomp.) (recrystallized from dimethylformamide-ethanol).

(2) N-(5-Tetrazolyl)-4-methyl-6-(4-N-methyl-N-benzyloxycarbonylaminophenyl)-2-pyridinecarboxamide (0.48 g) obtained in (1) is treated in the same manner as described in Example 8 — (2) to give N-(5-tetrazolyl)-4-methyl-6-(4-methylaminophenyl)-2-pyridinecarboxamide (0.32 g). M.P. 272°—273°C (decomp.) (wetted from 267°C) (recrystallized from dimethylformamide-ethanol).

Analysis calculated for $C_{15}H_{15}ON_7 \cdot 1/2\ C_2H_5OH$

C, 57.82; H, 5.46; N, 29.50

Found: C, 57.43; H, 5.51; N, 29.41

(3) N-(5-Tetrazolyl)-4-methyl-6-(4-methylaminophenyl)-2-pyridinecarboxamide obtained in (2) is treated in the same manner as described in Example 1 — (2) to give N-(5-tetrazolyl)-4-methyl-6-(4-methylaminophenyl)-2-pyridinecarboxamide sodium salt. This product is gradually decomposed at a temperature between 240°C and 270°C.

Example 10

(1) In the same manner as described in Example 1 — (1), 4-methoxy-6-(4-methyl-N-benzyloxycarbonylaminophenyl)-2-pyridinecarboxylic acid (1.19 g), carbonyldiimidazole (0.74 g) and 5-aminotetrazole (0.39 g) are reacted to give N-(5-tetrazolyl)-4-methoxy-6-(4-N-methyl-N-benzyloxycarbonylaminophenyl)-2-pyridinecarboxamide (1.17 g). M.P. 231°—232°C (decomp.).

(2) N-(5-Tetrazolyl)-4-methoxy-6-(4-N-methyl-N-benzyloxycarbonylaminophenyl)-2-pyridinecarboxamide (1.17 g) obtained in (1) is treated in the same manner as described in Example 8 — (2) to give N-(5-tetrazolyl)-4-methoxy-6-(4-methylaminophenyl)-2-pyridinecarboxamide (0.77 g). M.P. 251°—252°C (decomp.) (washed with ethanol).

(3) N-(5-Tetrazolyl)-4-methoxy-6-(4-methylaminophenyl)-2-pyridinecarboxamide obtained in (2) is treated in the same manner as described in Example 1 — (2) to give N-(5-tetrazolyl)-4-methoxy-6-(4-methylaminophenyl)-2-pyridinecarboxamide sodium salt. M.P. 210°—240°C (decomp.).

Example 11

(1) In the same manner as described in Example 1 — (1), 4-ethoxy-6-(4′-N-benzyloxycarbonylaminophenyl)-2-pyridinecarboxlic acid (1.34 g), carbonyldiimidazole (0.61 g) and 5-aminotetrazole (0.32 g) are reacted to give N-(5-tetrazolyl)-4-ethoxy-6-(4-N-benzyloxycarbonylaminophenyl)-2-pyridinecarboxamide (1.54 g). M.P. 235°—236°C (decomp.) (wetted at about 120°C).

(2) N-(5-Tetrazolyl)-4-ethoxy-6-(4-N-benzyloxycarbonylaminophenyl)-2-pyridinecarboxamide (1.54 g) obtained in (1) is treated in the same manner as described in Example 8 — (2) to give N-(5-tetrazolyl)-4-ethoxy-6-(4-aminophenyl)-2-pyridinecarboxamide (0.80 g). M.P. 263°—265°C (decomp.) (washed with ethanol).

(3) N-(5-Tetrazolyl)-4-ethoxy-6-(4-aminophenyl)-2-pyridinecarboxamide obtained in (2) is treated in the same manner as described in Example 1 — (2) to give N-(5-tetrazolyl)-4-ethoxy-6-(4-aminophenyl)-2-pyridinecarboxamide sodium salt. M.P. decomposed gradually from 247°C (wetted at about 220°C).

Example 12

(1) In the same manner as described in Example 1 — (1), 4-propoxy-6-(4-N-benzyloxycarbonylaminophenyl)-2-pyridinecarboxylic acid (1.33 g), carbonyldiimidazole (0.59 g) and 5-aminotetrazole (0.31 g) are reacted to give N-(5-tetrazolyl)-4-propoxy-6-(4-N-benzyloxycarbonylaminophenyl)-2-pyridinecarboxamide (1.30 g). M.P. 230°—231°C (decomp.).

(2) N-(5-Tetrazolyl)-4-propoxy-6-(4-N-benzyloxycarbonylaminophenyl)-2-pyridinecarboxamide (1.30 g) is treated in the same manner as described in Example 8 — (2) to give N-(5-tetrazolyl)-4-propoxy-6-(4-aminophenyl)-2-pyridinecarboxamide (0.33 g). M.P. 237°—240°C (decomp.) (washed with isopropyl alcohol).

(3) N-(5-Tetrazolyl)-4-propoxy-6-(4-aminophenyl)-2-pyridinecarboxamide obtained in (2) is treated in the same manner as described in Example 1 — (2) to give N-(5-tetrazolyl)-4-propoxy-6-(4-aminophenyl)-2-pyridinecarboxamide sodium salt. M.P. decomposed gradually from 245°C (wetted from 220°C).

Example 13

(1) A mixture of 6-(4-nitrophenyl)-2-pyridinecarboxylic acid (4.0 g), thionyl chloride (17 ml) and dimethylformamide (4 drops) is agitated at a bath temperature of 70° to 80°C for 8 hours. After the reaction, the reaction mixture is concentrated under reduced pressure. The resulting crude acid chloride is dissolved in dimethylformamide (60 ml) and thereto is added dropwise with agitation a solution of 5-aminotetrazole (1.54 g) and triethylamine (4.14 g) in dimethylformamide (15 ml). The mixture is agitated at room temperature for 20 hours, and the reaction mixture is concentrated under reduced pressure and thereto is added water. The mixture is made acidic with 10% hydrochloric acid, and the precipitated crystals are collected by filtration. The crystals thus obtained are washed with water and ethanol and dried to give N-(5-tetrazolyl)-6-(4-nitrophenyl)-2-pyridinecarboxamide (4.1 g). M.P. 303°—304°C (decomp.) (wetted from 294°C).

(2) A suspended mixture of N-(5-tetrazolyl)-6-(4-nitrophenyl)-2-pyridinecarboxamide (2.5 g) obtained in (1), 1N aqueous sodium hydroxide (10 ml), ethanol (90 ml), water (20 ml) and 10% palladium-carbon (0.8 g) is shaked under hydrogen gas at room temperature under atmospheric pressure for one hour and 45 minutes. After the reaction, the catalyst is removed by filtration and the filtrate is neutralized with 10% hydrochloric acid. After removing ethanol under reduced pressure, the resulting crystals are collected by filtration, washed with water and dried to give N-(5-tetrazolyl)-6-(4-aminophenyl)-2-pyridinecarboxamide (1.9 g). M.P. 268°—273°C (decomp.) (recrystallized from dimethylformamide-ethanol).

(3) N-(5-Tetrazolyl)-6-(4-aminophenyl)-2-pyridinecarboxamide obtained in (2) is treated in the same manner as described in Example 1 — (2) to give N-(5-tetrazolyl)-6-(4-aminophenyl)-2-pyridinecarboxamide sodium salt. M.P. changed to yellow from 290°C.

Example 14

(1) In the same manner as described in Example 1 — (1), 4-methoxy-6-(3-nitrophenyl)-2-pyridinecarboxylic acid (0.4 g), carbonyldiimidazole (0.22 g) and 5-aminotetrazole (0.15 g) are reacted to give N-(5-tetrazolyl)-4-methoxy-6-(3-nitrophenyl)-2-pyridinecarboxamide (0.31 g). M.P. 269°—272.5°C (decomp.) (wetted from about 250°C) (recrystallized from dimethylformamide-ethyl acetate).

(2) N-(5-Tetrazolyl)-4-methoxy-6-(3-nitrophenyl)-2-pyridinecarboxamide (1.34 g) obtained in (1) is treated in the same manner as described in Example 13 — (2) to give N-(5-tetrazolyl)-4-methoxy-6-(3-aminophenyl-2-pyridine-carboxamide (0.97 g). M.P. 259°—260°C (decomp.) (wetted from about 250°C) (recrystallized from dimethylformamide).

(3) N-(5-Tetrazolyl)-4-methoxy-6-(3-aminophenyl)-2-pyridinecarboxamide obtained in (2) is treated in the same manner as described in Example 1 — (2) to give N-(5-tetrazolyl)-4-methoxy-6-(3-aminophenyl)-2-pyridinecarboxamide sodium salt. M.P. 210°—215°C (decomp.) (wetted at about 200°C).

Example 15

(1) In the same manner as described in Example 13 — (1), 6-(3-nitrophenyl)-2-pyridinecarboxylic acid (4.0 g), thionyl chloride (12 ml) and 5-aminotetrazole (1.54 g) are reacted to give N-(5-tetrazolyl)-6-(3-nitrophenyl)-2-pyridinecarboxamide (4.9 g). M.P. 275°—279°C (decomp.).

(2) N-(5-Tetrazolyl)-6-(3-nitrophenyl)-2-pyridinecarboxamide (2.5 g) obtained in (1) is treated in the same manner as described in Example 13 — (2) to give N-(5-tetrazolyl)-6-(3-aminophenyl)-2-pyridinecarboxamide (1.93 g). M.P. 274°—275°C (decomp.) (recrystallized from dimethylformamide-ethanol).

(3) N-(5-Tetrazolyl)-6-(3-aminophenyl)-2-pyridinecarboxamide obtained in (2) is treated in the same manner as described in Example 1 — (2) to give N-(5-tetrazolyl)-6-(3-aminophenyl)-2-pyridinecarboxamide sodium salt. M.P. 238°—300°C (decomp.) (wetted from 223°C).

Example 16

(1) In the same manner as described in Example 13 — (1), 4-methyl-6-(4-nitrophenyl)-2-pyridinecarboxylic acid (3.0 g), thionyl chloride (20 ml) and 5-aminotetrazole (1.29 g) are reacted to give N-(5-tetrazolyl)-4-methyl-6-(4-nitrophenyl)-2-pyridinecarboxamide (2.5 g). M.P. 284°—285°C (decomp.) (wetted from 278°C) (recrystallized from dimethylformamide-ethanol).

(2) N-(5-Tetrazolyl)-4-methyl-6-(4-nitrophenyl)-2-pyridinecarboxamide (2.05 g) obtained in (1) is treated in the same manner as described in Example 13 — (2) to give N-(5-tetrazolyl)-4-methyl-6-(4-aminophenyl)-2-pyridinecarboxamide (1.45 g). M.P. 259.5°—260.5°C (decomp.) (wetted from 230°C) (recrystallized from dimethylformamide-ethanol).

(3) N-(5-Tetrazolyl)-4-methyl-6-(4-aminophenyl)-2-pyridinecarboxamide obtained in (2) is treated in the same manner as described in Example 1 — (2) to give N-(5-tetrazolyl)-4-methyl-6-(4-aminophenyl)-2-pyridinecarboxamide sodium salt. M.P. 278°—287°C (decomp.) (wetted from 246°C).

Example 17

(1) In the same manner as described in Example 13 — (1), 4-methyl-6-(3-nitrophenyl)-2-pyridinecarboxylic acid (3.0 g), thionyl chloride (30 ml) and 5-aminotetrazole (1.2 g) are reacted to given N-(5-tetrazolyl)-4-methyl-6-(3-nitrophenyl)-2-pyridinecarboxamide (1.92 g). M.P. 288°—290°C (decomp.) (recrystallized from dimethylformamide).

(2) N-(5-Tetrazolyl)-4-methyl-6-(3-nitrophenyl)-2-pyridinecarboxamide (1.15 g) obtained in (1) is treated in the same manner as described in Example 13 — (2) to give N-(5-tetrazolyl)-4-methyl-6-(3-aminophenyl)-2-pyridinecarboxamide hydrochloride (1.09 g). M.P. 266°—272°C (decomp.) (washed with ethanol).

Example 18

(1) In the same manner as described in Example 13 — (1), 4-isopropyl-6-(4-nitrophenyl)-2-pyridinecarboxylic acid (1.62 g), thionyl chloride (15 ml) and 5-aminotetrazole (0.63 g) are reacted to give N-(5-tetrazolyl)-4-isopropyl-6-(4-nitrophenyl)-2-pyridinecarboxamide (0.85 g). M.P. 270°—272°C (decomp.) (recrystallized from dimethylformamide-ethanol).

(2) N-(5-Tetrazolyl)-4-isopropyl-6-(4-nitrophenyl)-2-pyridinecarboxamide (0.85 g) obtained in (1) is treated in the same manner as described in Example 13 — (2) to give N-(5-tetrazolyl)-4-isopropyl-6-(4-aminophenyl)-2-pyridinecarboxamide (0.6 g). M.P. 268°—270°C (decomp.) (recrystallized from dimethylformamide-ethanol).

(3) N-(5-Tetrazolyl)-4-isopropyl-6-(4-aminophenyl)-2-pyridinecarboxamide obtained in (2) is treated in the same manner as described in Example 1 — (2) to give N-(5-tetrazolyl)-4-isopropyl-6-(4-aminophenyl)-2-pyridinecarboxamide sodium salt. M.P. 259—265°C (decomp.) (wetted from 241°C).

Example 19

(1) In the same manner as described in Example 13 — (1), 4-n-butyl-6-(4-nitrophenyl)-2-pyridinecarboxylic acid (1.7 g), thionyl chloride (15 ml) and 5-aminotetrazole (0.67 g) are reacted to give N-(5-tetrazolyl)-4-n-butyl-6-(4-nitrophenyl)-2-pyridinecarboxamide (1.16 g). M.P. 278°—280°C (decomp.) (recrystallized from dimethylformamide-ethanol).

(2) N-(5-Tetrazolyl)-4-n-butyl-6-(4-nitrophenyl)-2-pyridinecarboxamide (1.16 g) obtained in (1) is treated in the same manner as described in Example 13 — (2) to give N-(5-tetrazolyl)-4-n-butyl-6-(4-aminophenyl)-2-pyridinecarboxamide (0.64 g). M.P. >300°C (recrystallized from dimethylformamide-ethanol).

(3) N-(5-Tetrazolyl)-4-n-butyl-6-(4-aminophenyl-2-pyridinecarboxamide obtained in (2) is treated in the same manner as described in Example 1 — (2) to give N-(5-tetrazolyl)-4-n-butyl-6-(4-aminophenyl)-2-pyridinecarboxamide sodium salt. M.P. 248°—252°C (decomp.) (wetted from 243°C).

Example 20

(1) In the same manner as described in Example 13 — (1), 4-methoxy-6-(4-nitrophenyl)-2-pyridinecarboxylic acid (2.54 g), thionyl chloride (30 ml) and 5-aminotetrazole (1.2 g) are treated to give N-(5-tetrazolyl)-4-methoxy-6-(4-nitrophenyl)-2-pyridinecarboxamide (2.08 g). M.P. 279°—281°C (decomp.) (recrystallized from dimethylformamide).

(2) N-(5-Tetrazolyl)-4-methoxy-6-(4-nitrophenyl)-2-pyridinecarboxamide (1.7 g) obtained in (1) is treated in the same manner as described in Example 13 — (2) to give N-(5-tetrazolyl)-4-methoxy-6-(4-aminophenyl)-2-pyridinecarboxamide (0.14 g). M.P. 251°—253°C (decomp.) (washed with ethanol)

(3) N-(5-Tetrazolyl)-4-methoxy-6-(4-aminophenyl)-2-pyridinecarboxamide obtained in (2) is treated in the same manner as described in Example 1 — (2) to give N-(5-tetrazolyl)-4-methoxy-6-(4-aminophenyl)-2-pyridinecarboxamide sodium salt. M.P. 230°—234°C (decomp.).

Example 21

(1) In the same manner as described in Example 13 — (1), 4-chloro-6-(4-N-benzyloxycarbonylaminophenyl)-2-pyridinecarboxylic acid (0.94 g), thionyl chloride (8 ml) and 5-aminotetrazole (0.31 g) are reacted to give N-(5-tetrazolyl)-4-chloro-6-(4-N-benzyloxycarbonylaminophenyl)-2-pyridinecarboxamide (0.44 g). M.P. 251°—253°C (decomp.) (recrystallized from dimethylformamide-ethanol).

(2) A mixture of N-(5-tetrazolyl)-4-chloro-6-(4-N-benzyloxycarbonylaminophenyl)-2-pyridinecarboxamide (0.43 g) obtained in (1) and 25% hydrobromic acid-acetic acid solution (4 ml) is agitated at room temperature for 4 hours. To the reaction mixture is added ice water, the precipitated crystals are collected by filtration and washed with water, ethanol and ether to give faint yellowish green powder of N-(5-tetrazolyl)-4-chloro-6-(4-aminophenyl)-2-pyridinecarboxamide (0.19 g). M.P. decomposed gradually with coloring from 200°C.

(3) N-(5-Tetrazolyl)-4-chloro-6-(4-aminophenyl)-2-pyridinecarboxamide obtained in (2) is treated in the same manner as described in Example 1 — (2) to give N-(5-tetrazolyl)-4-chloro-6-(4-aminophenyl)-2-pyridinecarboxamide sodium salt. M.P. colored gradually from 230°C.

Reference Examples 1 to 6

4-Methyl-2-phenylpyridine nitrate prepared from 4-methyl-2-phenylpyridine (26.3 g) and 69% nitric acid (15 ml) is added under cooling to conc. sulfuric acid (95 ml), and the mixture is heated with agitation at 90°C—100°C for 30 minutes. After cooling, ice water (300 ml) is added to the reaction mixture, and the mixture is adjusted to pH 3.5 with conc. aqueous ammonia. The crystals precipitated from the hot solution are collected by filtration and recrystallized from ethanol to give 4-methyl-2-(4-nitrophenyl)pyridine (13.3 g). M.P. 145°—147°C.

Besides, the above mother liquor is cooled, and the precipitated crystals are collected by filtration and recrystallized from ethanol to give 4-methyl-2-(3-nitrophenyl)pyridine (9.3 g). M.P. 80°—82°C

In the same manner as described above, the compounds as shown in Table 2 are prepared.

TABLE 2

| 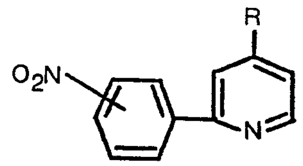 | | | |
|---|---|---|---|
| Ref. Ex. No. | 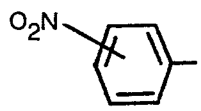 | R | M.P. (°C) |
| 3 | 4-Nitrophenyl | Hydrogen | 126—128 |
| 4 | 3-Nitrophenyl | Hydrogen | 68—74 |
| 5 | 4-Nitrophenyl | Isopropyl | 156—158 (picrate) |
| 6 | 4-Nitrophenyl | n-Butyl | 177—180 (picrate) |

Reference Examples 7 to 12

(1) A mixture of 4-methyl-2-(4-nitrophenyl)pyridine (25.8 g), acetic acid (130 ml) and 30% hydrogen peroxide (27.5 ml) is agitated at 75°—85°C for 17 hours. To the mixture is added 10% palladium-carbon in order to decompose excess amount of hydrogen peroxide, and then the palladium-carbon is removed by filtration. The filtrate is concentrated, and the concentrated solution is made alkaline with potassium carbonate and extracted with ethyl acetate. The extract is washed with water and dried, and ethyl acetate is distilled off to give 4-methyl-2-(4-nitrophenyl)pyridine N-oxide (26.6 g). M.P. 158°—160°C.

(2) A mixture of 4-methyl-2-(4-nitrophenyl)pyridine N-oxide (26.5 g) obtained in (1), dimethyl sulfate (15.2 g) and dioxane (30 ml) is agitated at 80°C for 2 hours to give the corresponding N-methoxypyridinium salt. To the reaction mixture is added dioxane-water (7:3) (70 ml) and is further added a solution of sodium cyanide (16.9 g) in water (100 ml) at 5°C and thereto are further added dioxane (20 ml) and water (50 ml). The mixture is agitated at 5°C for 30 minutes and further at room temperature for 1.5 hour. The precipitated crystals are collected by filtration, washed with water and dried to give 2-cyano-4-methyl-6-(4-nitrophenyl)pyridine (25 g). M.P. 146°—156°C.

(3) A mixture of 2-cyano-4-methyl-6-(4-nitrophenyl)pyridine (24.5 g) obtained in (2) and conc. hydrochloric acid (240 ml) is agitated at 110°—120°C for 16 hours. After the reaction, the reaction mixture is concentrated under reduced pressure and is adjusted to about pH 2 with aqueous sodium hydroxide. The precipitated crystals are collected by filtration, washed with water and dried to give 4-methyl-6-(4-nitrophenyl)-2-pyridinecarboxylic acid (24 g). M.P. 216°—219°C.

In the same manner as described above, the compounds as shown in Table 3 are prepared.

TABLE 3

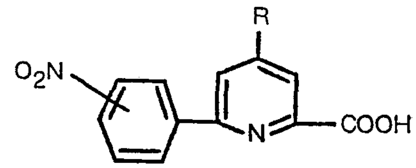

| Ref. Ex. No. | 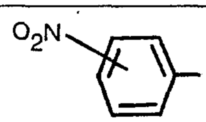 | R | M.P. (°C) |
|---|---|---|---|
| 8 | 3-Nitrophenyl | Hydrogen | 224—225 |
| 9 | 4-Nitrophenyl | Hydrogen | 178—179 |
| 10 | 3-Nitrophenyl | Methyl | 237—241 (decomp.) |
| 11 | 4-Nitrophenyl | Isopropyl | 138—144 |
| 12 | 4-Nitrophenyl | n-Butyl | 112—115 |

Reference Examples 13 to 14

A mixture of 4-methyl-6-(4-nitrophenyl)-2-pyridinecarboxylic acid (19.1 g), 10% sodium hydroxide (50 ml), water (200 ml), ethanol (200 ml) and 10% palladium-carbon (3 g) is shaken under hydrogen gas stream at room temperature under atmospheric pressure. After the reduction is finished, the catalyst is removed by filtration. The filtrate is made acidic with diluted hydrochloric acid and concentrated under reduced pressure. To the resulting residue are added ethanol (800 ml) and thionyl chloride (40 ml), and the mixture is heated under reflux for 4 hours. After treating with charcoal, the reaction mixture is concentrated under reduced pressure in order to distill off the solvent. The resulting residue is recrystallized from ethanol-ether to give ethyl 4-methyl-6-(4-aminophenyl)-2-pyridinecarboxylate hydrochloride (19.4 g). M.P. 210°—213°C (decomp.).

In the same manner as described above, the following compound is prepared.

Methyl 6-(4-aminophenyl)-2-pyridinecarboxylate, M.P. 116°—119°C

Reference Examples 15 to 16

(1) A mixture of ethyl 4-methyl-6-(4-aminophenyl)-2-pyridinecarboxylate hydrochloride (3.5 g), anhydrous potassium carbonate (8.8 g), dimethylformamide (30 ml) and methyl iodide (10 ml) is agitated at 50°C for 30 minutes and further at 130°C for 3 hours. After cooling, the reaction mixture is filtered to remove potassium carbonate. The filtrate is concentrated under reduced pressure in order to distill off the solvent. To the resulting residue is added water, and the mixture is extracted with ethyl acetate. The extract is washed with water and dried, and ethyl acetate is distilled off to give ethyl 4-methyl-6-(4-dimethylaminophenyl)-2-pyridinecarboxylate (2.2 g). M.P. 143°—144°C.

(2) Ethyl 4-methyl-6-(4-dimethylaminophenyl)-2-pyridinecarboxylate (2.2 g) obtained in (1) is mixed with 10% aqueous sodium hydroxide (6 ml) and methanol (20 ml), and the mixture is agitated at 50°—60°C for 4 hours. The reaction mixture is concentrated under reduced pressure and thereto is added water, and made acidic with 10% hydrochloric acid and concentrated under reduced pressure to give crude 4-methyl-6-(4-dimethylaminophenyl)-2-pyridinecarboxylic acid hydrochloride (2.5 g).

In the same manner as described above, the following compound is prepared.

4-Methyl-6-(4-diethylaminophenyl)-2-pyridinecarboxylic acid, M.P. (hydrochloride) 223°—225°C (decomp.)

Reference Examples 17 to 18

(1) To a mixture of ethyl 4-methyl-6-(4-aminophenyl)-2-pyridinecarboxylate hydrochloride (6 g), water (150 ml), sodium hydrogen carbonate (6 g) and ethyl acetate (150 ml) is added under ice-cooling with agitation benzyloxycarbonyl chloride (4.2 g), and the mixture is agitated for 45 minutes. The reaction mixture is made acidic with diluted hydrochloric acid, and the resulting crystals are collected by filtration, washed with water and dried. The organic layer is separated from the filtrate, washed with water and dried, and then the solvent is distilled off therefrom under reduced pressure. To the resulting residue is added ethyl acetate, and the precipitated crystals are collected by filtration and combined with the crystals obtained above to give ethyl 4-methyl-6-(4-benzyloxycarbonylaminophenyl)-2-pyridinecarboxylate (6 g). M.P. 184°—185°C.

(2) A mixture of ethyl 4-methyl-6-(4-benzyloxycarbonylaminophenyl)-2-pyridinecarboxylate (6 g) obtained in (1), potassium carbonate (18 g), dimethylformamide (50 ml) and methyl iodide (15 ml) is agitated at room temperature for 20 hours. After the reaction, potassium carbonate is removed by filtration, and the filtrate is concentrated under reduced pressure. To the resulting residue is added water, and the mixture is extracted with ethyl acetate. After drying, the extract is distilled to remove the solvent, and thereto is added a small amount of ethyl acetate, and insoluble materials are removed by filtration. The filtrate is concentrated under reduced pressure in order to remove the solvent to give ethyl 4-methyl-6-(4-N-methyl-N-benzyloxycarbonylaminophenyl)-2-pyridinecarboxylate (2.7 g) as an oily substance.

(3) A mixture of ethyl 4-methyl-6-(4-N-methyl-N-benzyloxycarbonylaminophenyl)-2-pyridinecarboxylate (2.7 g) obtained in (2), 10% aqueous sodium hydroxide (5 ml) and ethanol (40 ml) is agitated at room temperature for 20 hours. After ethanol is distilled off, water is added to the reaction mixture, and the mixture is made acidic with 10% hydrochloric acid. The mixture is extracted with ethyl acetate. The extract is washed with water, dried and then the solvent is distilled off to give 4-methyl-6-(4-N-methyl-N-benzyloxycarbonylaminophenyl)-2-pyridinecarboxylic acid (2.4 g) as an oily substance.

In the same manner as described above, the following compound is prepared.

6-(4-N-Methyl-N-benzyloxycarbonylaminophenyl)-2-pyridinecarboxylic acid, M.P. 114°—115°C

Reference Example 19

(1) A free base of ethyl 4-methyl-6-(4-aminophenyl)-2-pyridinecarboxylate obtained from its hydrochloride salt (2 g) is added to chloroform (10 ml) and acetic anhydride (10 ml), and the mixture is allowed to stand at room temperature for 2 days. The precipitated crystals are collected by filtration and washed with ether. Besides, the filtrate is concentrated under reduced pressure to remove the solvent, and to the residue is added ether. The precipitated crystals are collected by filtration and combined with the crystals obtained above to give ethyl 4-methyl-6-(4-acetylaminophenyl)-2-pyridinecarboxylate (1.6 g). M.P. 224°—225°C (decomp.).

(2) A mixture of ethyl 4-methyl-6-(4-acetylaminophenyl)-2-pyridinecarboxylate (1.3 g) obtained in (1), methanol (150 ml) and 10% aqueous sodium hydroxide (6.5 ml) is agitated at 50°—60°C for 14 hours. The reaction mixture is concentrated under reduced pressure and is made acidic with hydrochloric acid. The precipitate crystals are collected by filtration, washed with water and dried to give 4-methyl-6-(4-acetylaminophenyl)-2-pyridinecarboxylic acid (1.12 g). M.P. 253°—254°C (decomp).

Reference Examples 20 to 21

(1) Ethanol (9.5 ml) is added to a suspension of 60% sodium hydride (26.3 g) in anhydrous ether (750 ml), and thereto is added dropwise a mixture of p-nitrobenzalacetone (130 g), diethyl oxalate (99.4 g) and anhydrous tetrahydrofuran (550 ml) at 5—10°C with agitation. The mixture is agitated at room temperature for one hour, and thereto is added 10% hydrochloric acid (350 ml). The precipitated yellow solid is collected by filtration, washed with ether and then recrystallized from acetone to give ethyl 6-(4-nitrophenyl)-2,4-dioxo-hex-5-enoate (87.8 g). M.P. 148.5°—153.5°C.

(2) Ethyl 6-(4-nitrophenyl-2,4-dioxo-hex-5-enoate (30.0 g) is dissolved in methylene chloride (200 ml) and thereto is added dropwise a solution of bromine (5.8 ml) in methylene chloride (100 ml) at 0° to −5°C over a period of 30 minutes. The mixture is agitated at the same temperature and then distilled under reduced pressure to remove the solvent. To the resulting oily residue [ethyl 6-(4-nitrophenyl)-5,6-dibromo-2,4-dioxo-hexanoate] are added potassium acetate (25 g) and ethyl alcohol (250 ml), and the mixture is agitated at 50°—60°C for 2 hours. The mixture is concentrated under reduced pressure to remove the solvent, and thereto is added water. The insoluble solid materials are collected by filtration and dissolved in chloroform with heating. After removing the insoluble materials by filtration, the solution is distilled. The resulting residue is recrystallized from ethanol to give ethyl 6-(4-nitrophenyl)-4H-pyran-4-one-2-carboxylate (7.23 g). M.P. 158°—159°C.

In the same manner as described above, the following compound is prepared.

Ethyl 6-(3-nitrophenyl)-4H-pyran-4-one-2-carboxylate, M.P. 152°—154°C.

Reference Examples 22 to 23

A mixture of ethyl 6-(4-nitrophenyl)-4H-pyran-4-one-2-carboxylate (11.03 g) and 14% ammonia-ethanol (300 ml) is heated in a pressure vessel at 120°—140°C for 42 hours. After cooling, the mixture is distilled under reduced pressure to remove ethanol. The resulting crystals are washed with ethanol to give 4-hydroxy-6-(4-nitrophenyl)-2-pyridinecarboxamide (7.25 g. M.P. >300°C.

In the same manner as described above, the following compound is prepared.

4-Hydroxy-6-(3-nitrophenyl)-2-pyridinecarboxamide, M.P. 303°—305°C (decomp.).

Reference Examples 24 to 25

(1) A mixture of 4-hydroxy-6-(3-nitrophenyl)-2-pyridinecarboxamide (1 g) and conc. hydrochloric acid (150 ml) is heated under reflux for 3 hours. The reaction mixture is concentrated under reduced pressure, and to the residue is added water. The mixture is adjusted to about pH 2 with sodium

hydrogen carbonate, and the precipitated crystals are collected by filtration to give 4-hydroxy-6-(3-nitrophenyl)-2-pyridinecarboxylic acid (0.9 g). M.P. 263°—264°C (decomp.).

(2) A mixture of 4-hydroxy-6-(3-nitrophenyl)-2-pyridinecarboxylic acid (0.52 g) obtained in (1), dimethylformamide (7 ml), water (2 ml), potassium carbonate (1 g) and methyl iodide (1.5 ml) is agitated at 80°C for 2 hours. The reaction mixture is concentrated under reduced pressure, and to the residue is added water. The precipitated crystals are collected by filtration to give methyl 4-methoxy-6-(3-nitrophenyl)-2-pyridinecarboxylate (0.52 g). M.P. 150°—152°C.

(3) A mixture of methyl 4-methoxy-6-(3-nitrophenyl)-2-pyridinecarboxylate (0.47 g) obtained in (2) and 5% potassium hydroxide-methanol (20 ml) is agitated at 70°C for several minutes, and thereto is added water. The mixture is made acidic with 10% hydrochloric acid. The precipitated crystals are collected by filtration to give 4-methoxy-6-(3-nitrophenyl)-2-pyridinecarboxylic acid (0.42 g). M.P. 207°—210°C.

In the same mannse as described above, the following compound is prepared.

4-Methoxy-6-(4-nitrophenyl)-2-pyridinecarboxylic acid, M.P. 208°—211°C.

Reference Example 26

A mixture of ethyl 6-(4-nitrophenyl)-4H-pyran-4-one-2-carboxylate (59 g), 10% palladium-carbon (6 g), potassium carbonate (10 g) and dimethylformamide (400 ml) is shaken under hydrogen gas stream at room temperature under atmospheric pressure. After the reduction reaction is finished, the insuluble materials are removed by filtration, and thereto is added water. The precipitated crystals are collected by filtration to give ethyl 6-(4-aminophenyl)-4H-pyran-4-one-2-carboxylate (30 g). M.P. 190°—193°C.

Reference Example 27

(1) A mixture of ethyl 6-(4-aminophenyl)-4H-pyran-4-one-2-carboxylate (24 g) and 13% ammonia-ethanol (400 ml) is heated in a pressure vessel at 120°C for 60 hours. After the reaction, the mixture is distilled to remove ethanol, and to the resulting residue is added ether. The precipitated crystals are collected by filtration to give 4-hydroxy-6-(4-aminophenyl)-2-pyridinecarboxamide (20.9 g). M.P. 251°—253°C (decomp.).

(2) A mixture of 4-hydroxy-6-(4-aminophenyl)-2-pyridinecarboxamide (20.9 g) obtained in (1), 5N aqueous sodium hydroxide (300 ml) and ethanol (150 ml) is agitated at 100°C for 2 hours. After the reaction, the reaction mixture is concentrated under reduced pressure, and to the residue is added methanol (400 ml). The mixture is heated under reflux for 3 hours while introducing hydrochloric acid gas thereto. The mixture is further heated under reflux for 14 hours, and then methanol is distilled off. To the resulting residue is added water, and the mixture is neutralized with powdery sodium hydrogen carbonate. The precipitated crystals are collected by filtration and washed with water to give methyl 4-hydroxy-6-(4-aminophenyl)-2-pyridinecarboxylate (11.4 g). M.P. 188°—190°C (decomp.).

Reference Examples 28 to 30

(1) A mixture of methyl 4-hydroxy-6-(4-aminophenyl)-2-pyridinecarboxylate (1.22 g), methyl iodide (3.82 g), potassium carbonate (2.76 g), dimethylformamide (20 ml) and water (1 ml) is agitated at room temperature for 3 hours. The reaction mixture is concentrated under reduced pressure, and to the residue is added water. The precipitation crystals are collected by filtration and dissolved in chloroform. The solution is passed through a column packed with silica gel (30 g). The solution passed through the column is concentrated to remove the solvent to give methyl 4-methoxy-6-(4-dimethylaminophenyl)-2-pyridinecarboxylate (0.74 g). M.P. 157°—158°C.

(2) A mixture of methyl 4-methoxy-6-(4-dimethylaminophenyl)-2-pyridinecarboxylate (0.82 g) obtained in (1), 10% aqueous sodium hydroxide (2 ml), tetrahydrofuran (15 ml) and methanol (5 ml) is agitated at room temperature for 30 minutes. The reaction mixture is concentrated under reduced pressure, and the residue is dissolved in water and adjusted to about pH 4 with 10% hydrochloric acid. The precipitated crystals are collected by filtration, washed with water and dried to give 4-methoxy-6-(4-dimethylaminophenyl)-2-pyridinecarboxylic acid (0.78 g). M.P. 142°—148°C.

In the same manner as described above, the following compounds are prepared.

4-Ethoxy-6-(4-diethylaminophenyl)-2-pyridinecarboxylic acid.

4-Ethoxy-6-(4-ethylaminophenyl)-2-pyridinecarboxylic acid.

Reference Example 31

To a mixture of methyl 4-hydroxy-6-(4-aminophenyl)-2-pyridinecarboxylate (2.4 g), sodium hydrogen carbonate (1.7 g), water (20 ml) and tetrahydrofuran (50 ml) is added dropwise under ice-cooling a solution of benzyloxycarbonyl chloride (1.7 g) in tetrahydrofuran (20 ml). The mixture is agitated at room temperature for 1.5 hour, and the mixture is neutralized with 10% hydrochloric acid. After concentrated under reduced pressure, the mixture is extracted with chloroform. The extract is dried and then chloroform is distilled off to give methyl 4-hydroxy-6-(4-N-benzyloxycarbonylaminophenyl)-2-pyridinecarboxylate (2 g). M.P. 204°—205°C (decomp.).

### Reference Example 32 to 33

(1) A mixture of methyl 4-hydroxy-6-(4-N-benzyloxycarbonylaminophenyl)-2-pyridinecarboxylate (1.89 g), ethyl iodide (1.2 g), potassium carbonate (0.69 g), dimethylformamide (20 ml) and water (1 ml) is agitated at room temperature for 17 hours. The reaction mixture is concentrated under reduced pressure and thereto is added water. The precipitated crystals are collected by filtration, washed with water and dried to give methyl 4-ethyl-6-(4-N-benzyloxycarbonylaminophenyl)-2-pyridinecarboxylate (1.97 g). M.P. 174°—176°C.

(2) Methyl 4-ethoxy-6-(4-N-benzylcarbonylaminophenyl)-2-pyridinecarboxylate (1.48 g) is added to a mixture of tetrahydrofuran (20 ml), methanol (6 ml) and 10% aqueous sodium hydroxide (3 ml), and the mixture is agitated at room temperature for 2.5 hours. The reaction mixture is concentrated under reduced pressure and thereto is added water. The mixture is adjusted to about pH 4 with 10% hydrochloric acid. The precipitated crystals are collected by filtration, washed with water and dried to give 4-ethoxy-6-(4-N-benzyloxycarbonylaminophenyl)-2-pyridinecarboxylic acid (1.34 g). M.P. softened gradually from 82°C.

In the same manner as described above, the following compound is prepared.

4-Propoxy-6-(4-N-benzyloxycarbonylaminophenyl)-2-pyridinecarboxylic acid.

### Reference Example 34

(1) To a solution of methyl 4-hydroxy-6-(4-N-benzyloxycarbonylaminophenyl)-2-pyridinecarboxylate (1.51 g) in dimethylformamide (20 ml) is added under ice-cooling sodium hydride (62% oily suspension) (0.46 g), and the mixture is agitated at room temperature for one hour. To the mixture is added under ice-cooling a solution of methyl iodide (3.06 g) in dimethylformamide (5 ml), and the mixture is agitated at room temperature for 2 hours. The reaction mixture is concentrated under reduced pressure and thereto is added water. The mixture is extracted with ethyl acetate. The extract is washed with water and dried. After distilled off the solvent, the resulting residue is recrystallized from isopropyl ether to give methyl 4-methoxy-6-(4-N-methyl-N-benzyloxycarbonylaminophenyl)-2-pyridinecarboxylate (1.30 g). M.P. 79°—83°C.

(2) Methyl 4-methoxy-6-(4-N-methyl-N-benzyloxycarbonylaminophenyl)-2-pyridinecarboxylate (1.30 g) obtained in (1) is added to a mixture of tetrahydrofuran (10 ml), methanol (3 ml) and 10% aqueous sodium hydroxide (3 ml), and the mixture is agitated at room temperature for 2.5 hours. The reaction mixture is concentrated under reduced pressure, and thereto is added water. The mixture is adjusted to about pH 4 with 10% hydrochloric acid, and the precipitated crystals are collected by filtration, washed with water and dried to give yellow caramel-like 4-methoxy-6-(4-N-methyl-N-benzyloxycarbonylaminophenyl)-2-pyridinecarboxylic acid (1.19 g). M.P. softened gradually from 60°C.

### Reference Example 35

(1) To a mixture of methyl 4-hydroxy-6-(4-aminophenyl)-2-pyridinecarboxylate (1.03 g) and acetic anhydride (15 ml) are added under ice-cooling pyridine (0.5 ml) and further tetrahydrofuran (20 ml), and the mixture is heated under reflux for 3 hours. The reaction mixture is concentrated under reduced pressure and thereto is added water. The precipitated crystals are collected by filtraton and washed with water and ether to give the corresponding N,O-diacetyl derivative. The reaction mixture is added to a mixture of methanol (30 ml) and 10% aqueous sodium hydroxide (10 ml), and the mixture is agitated at room temperature for 4.5 hours. The mixture is adjusted to about pH 3 with 10% hydrochloric acid and then concentrated under reduced pressure. The precipitated crystals are collected by filtration and washed with water to give the corresponding hydroxycarboxylic acid. This compound is added to a mixture of methyl iodide (3.22 g), potassium carbonate (2.9 g), dimethylformamide (30 ml) and water (1 ml), and the mixture is agitated at room temperature for 14 hours. The reaction mixture is concentrated under reduced pressure and thereto is added water. The precipitated crystals are collected by filtration to give methyl 4-methoxy-6-(4-acetylaminophenyl)-2-pyridinecarboxylate (0.80 g). M.P. 223°—224°.

(2) Methyl 4-methoxy-6-(4-acetylaminophenyl)-2-pyridinecarboxylate obtained in (1) is hydrolyzed with sodium hydroxide in usual manner to give 4-methoxy-6-(4-acetylaminophenyl)-2-pyridinecarboxylic acid (0.47 g). M.P. 234°—235°C (decomp.).

### Reference Example 36

(1) A mixture of methyl 4-hydroxy-6-(4-N-benzyloxycarbonylaminophenyl)-2-pyridinecarboxylate (1 g), thionyl chloride (10 ml) and dimethylformamide (0.4 ml) is agitated at 60°C for 30 minutes. The reaction mixture is concentrated under reduced pressure, and thereto is added water under ice-cooling. The precipitated crystals are collected by filtration, washed with water and dried to give methyl 4-chloro-6-(4-N-benzyloxycarbonylaminophenyl)-2-pyridinecarboxylate (0.89 g). M.P. 191°—193°C.

(2) Methyl 4-chloro-6-(4-N-benzyloxycarbonylaminophenyl)-2-pyridinecarboxylate (0.89 g) obtained in (1) is added to a mixture of 10% aqueous sodium hydroxide (2 ml), tetrahydrofuran (15 ml) and methanol (5 ml), and the mixture is agitated at room temperature for 15 minutes. The reaction mixture is concentrated under reduced pressure and thereto is added water. The mixture is made acidic

with 10% hydrochloric acid. The precipitated crystals are collected by filtation, washed with water and dried to give 4-chloro-6-(4-N-benzyloxycarbonylaminophenyl)-2-pyridinecarboxylic acid (0.84 g). M.P. 181°—183°C (decomp.).

## Claims

1. An N-(5-tetrazolyl)-6-phenyl-2-pyridinecarboxamide derivative of the formula

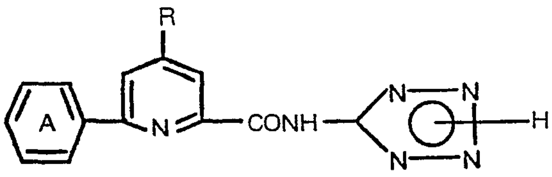

[I]

wherein R is hydrogen, a halogen, an alkyl($C_{1-4}$) or an alkoxy($C_{1-3}$), and the phenyl ring A is a phenyl having a substituent selected from amino, a mono-alkyl($C_{1-2}$)-amino, a di-alkyl($C_{1-2}$)-amino, and acetylamino, or a pharmaceutically acceptable salt thereof.

2. A compound according to claim 1, wherein R is selected from hydrogen, chlorine, methyl, ethyl, propyl, isopropyl, butyl, methoxy, ethoxy, propoxy and isopropoxy.

3. A compound according to claim 1, wherein the phenyl ring A is selected from 3-aminophenyl, 4-aminophenyl, 3-methylaminophenyl, 4-methylaminophenyl, 3-ethylaminophenyl, 4-ethylaminophenyl, 3-dimethylaminophenyl, 4-dimethylaminophenyl, 3-diethylaminophenyl, 4-diethylaminophenyl, 3-acetylaminophenyl, and 4-acetylaminophenyl.

4. A compound according to claim 1, which is N-(5-tetrazolyl)-4-methyl-6-(4-methylaminophenyl)-2-pyridine-carboxamide or a pharmaceutically acceptable salt thereof.

5. A process for the preparation of an N-(5-tetrazolyl)-6-phenyl-2-pyridinecarboxamide derivative of the formula

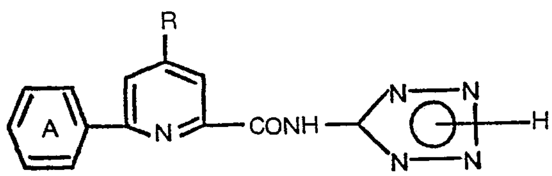

wherein R is hydrogen, a halogen, an alkyl($C_{1-4}$) or alkoxy($C_{1-3}$), and the phenyl ring A is a phenyl having a substituent selected from amino, a mono-alkyl($C_{1-2}$)-amino, a di-alkyl($C_{1-2}$)-amino, and acetylamino, or a pharmaceutically acceptable salt thereof, which comprises condensing a 6-phenyl-2-pyridinecarboxylic acid derivative of the formula:

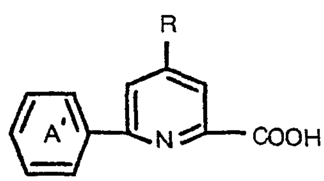

wherein R is as defined above, and the phenyl ring A′ is a phenyl having a substituent selected from nitro, a di-alkyl($C_{1-2}$)-amino, acetylamino, an amino protected with an amino-protecting group, and a mono-alkyl($C_{1-2}$)-amino protected with an amino-protecting group with 5-aminotetrazole, when the phenyl ring A′ is a phenyl having nitro substituent, said resulting compound being reduced in order to convert the nitro group into amino group, and when the phenyl ring A′ is a phenyl having a substituent selected from an amino protected with an amino-protecting group and a mono-alkyl($C_{1-2}$)-amino protected with an amino-protecting group, said amino-protecting group being removed from the resulting compound.

6. A process according to claim 5, wherein an N-(5-tetrazolyl)-6-phenyl-2-pyridinecarboxamide derivative of the formula

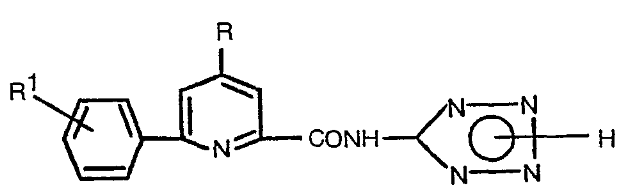

wherein R is hydrogen, a halogen, an alkyl($C_{1-4}$) or an alkoxy($C_{1-3}$), and $R^1$ is a di-alkyl($C_{1-2}$)-amino or acetylamino, is prepared by condensing a 6-phenyl-2-pyridinecarboxylic acid derivative of the formula:

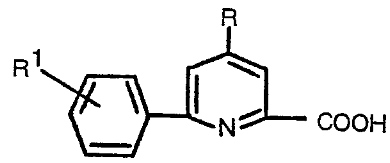

wherein R and $R^1$ are as defined above, with 5-aminotetrazole.

7. A process according to claim 5, wherein an N-(5-tetrazolyl)-6-phenyl-2-pyridinecarboxamide derivative of the formula

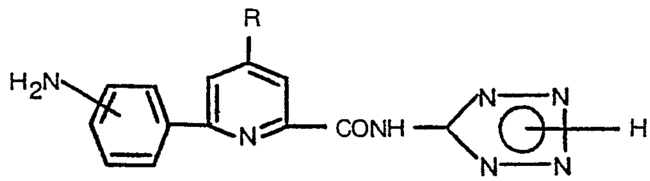

wherein R is hydrogen, a halogen, an alkyl($C_{1-4}$) or an alkoxy($C_{1-3}$), is prepared by condensing a 6-phenyl-2-pyridinecarboxylic acid derivative of the formula:

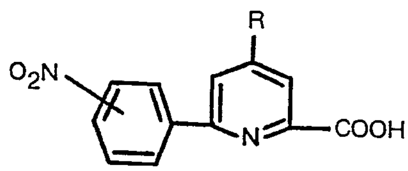

wherein R is as defined above, with 5-aminotetrazole to give a compound of the formula:

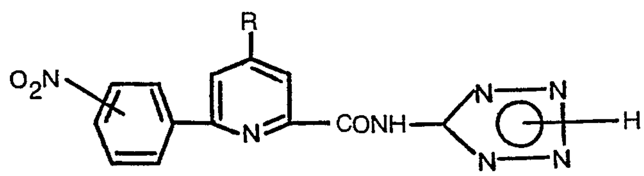

wherein R is as defined above, and reducing said compound in order to convert the nitro group into amino group.

8. A process according to claim 5, wherein an N-(5-tetrazolyl)-6-phenyl-2-pyridinecarboxamide derivative of the formula:

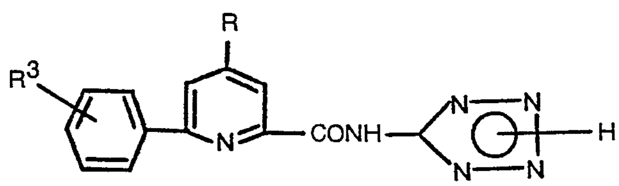

wherein R is hydrogen, a halogen, an alkyl($C_{1-4}$) or an alkoxy ($C_{1-3}$), and $R^3$ is amino or a mono-alkyl($C_{1-2}$)amino, is prepared by condensing 6-phenyl-2-pyridinecarboxylic acid derivative of the formula:

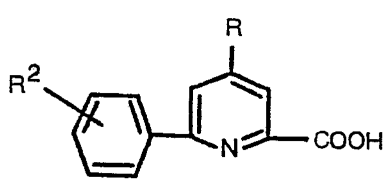

wherein R is as defined above, and $R^2$ is an amino protected with an amino-protecting group or a mono-

alkyl($C_{1-2}$)-amino protected with an amino-protecting group, with 5-aminotetrazole to give a compound of the formula:

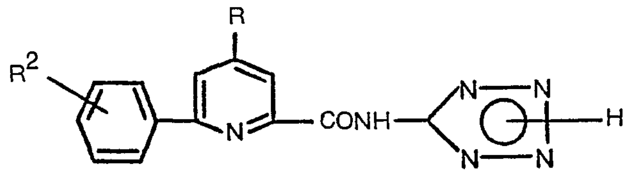

wherein R and $R^2$ are as defined above, and removing the amino-protecting group from the resulting compound.

9. A pharmaceutical composition which comprises an N-(5-tetrazolyl)-6-phenyl-2-pyridinecarboxamide derivative of the formula:

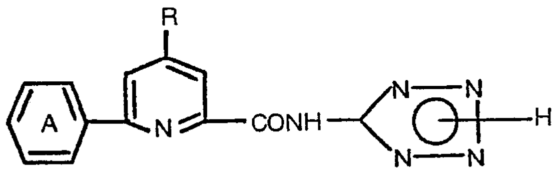

wherein R is hydrogen, a halogen, an alkyl($C_{1-4}$) or an alkoxy($C_{1-3}$), and the phenyl ring A is a phenyl having a substituent selected from amino, a mono-alkyl($C_{1-2}$)-amino, a di-alkyl($C_{1-2}$)-amino, and acetylamino, or a pharmaceutically acceptable salt thereof, and a pharmaceutically acceptable carrier therefor.

**Revendications**

1. Un dérivé du N-(5-tétrazolyl)-6-phényl-2-pyridinecarboxamide de la formule

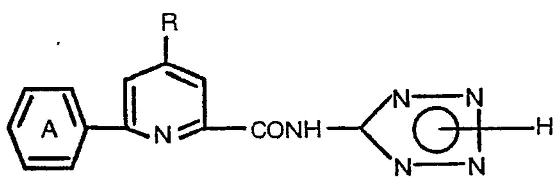

[I]

dans laquelle R est de l'hydrogène, un halogène, un alkyle (en $C_1$ à $C_4$) ou un alcoxy (en $C_1$ à $C_3$), et le noyau phényle est un phényle ayant un substituant choisi parmi un amino, un mono-alkyl (en $C_1$ à $C_2$)-amino, un di-alkyle (en $C_1$ à $C_2$)-amino, et un acétylamino, ou un de ses sels pharmaceutiquement acceptables.

2. Un composé selon la revendication 1, dans lequel R est choisi parmi l'hydrogène, le chlore, le méthyle, l'éthyle, le propyle, l'isopropyle, le butyle, le méthoxy, l'éthoxy, le propoxy et l'isopropoxy.

3. Un composé selon la revendication 1, dans lequel le noyau phénylique A est choisi parmi les 3-amino-phényle, 4-aminophényle, 3-méthylaminophényle, 4-méthylaminophényle, 3-éthylaminophényle, 4-éthylaminophényle, 3-diméthylaminophényle, 4-diméthylaminophényle, 3-diéthylaminophényle, 4-diéthylaminophényle, 3-acétylaminophényle et le 4-acétylaminophényle.

4. Un composé selon la revendication 1, qui est le N-(5-tétrazolyl)-4-méthyl-6-(4-méthylamino-phényl)-2-pyridine-carboxamide) ou un de ses sels pharmaceutiquement acceptables.

5. Un procédé pour le préparation d'un dérivé du N-(5-tétrazolyl)-6-phényl-2-pyridinecarboxamide de formule

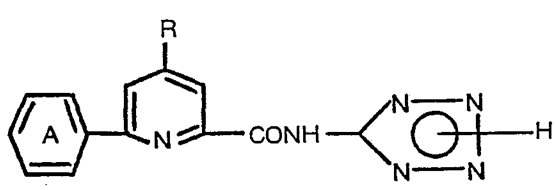

dans laquelle R est de l'hydrogène, un halogène, un alkyle (en $C_1$ à $C_4$) ou un alcoxy (en $C_1$ à $C_3$), et le noyau phénylique A est un phényle présentant un substituant choisi parmi un amino, un mono-alkyle (en $C_1$ à $C_2$)-amino, un di-alkyle (en $C_1$ à $C_2$)-amino, et un acétyl-amino, ou un de ses sels pharmaceutiquement acceptables, et qui consistent à condenser un dérivé de l'acide 6-phényle-2-pyridinecarboxylique de formule:

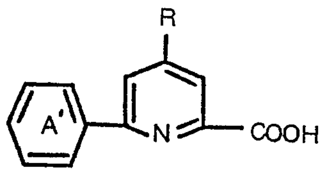

dans laquelle R est tel que défini ci-dessus, et le noyau phénylique A' est un phényle présentant un substituant choisi parmi un nitro, un di-alkyl (en $C_1$ à $C_2$)-amino, un acétylamino, un amino protégé par un groupe amino-protecteur, et un mono-alkyle (en $C_1$ à $C_2$)-amino protégé par un groupe amino-protecteur avec du 5-amino-tétrazole, lorsque le noyau phénylique A' est un phényle présentant un substituant nitro, ce composé résultant étant réduit afin de convertir le groupe nitro en groupe amino, et lorsque le noyau phénylique A' est un phényle présentant un substituant choisi parmi un amino protégé avec un groupe amino-protecteur et un mono-alkyle (en $C_1$ à $C_2$)-amino protégé avec un groupe amino-protecteur, ce groupe amino-protecteur étant éliminé du composé résultant.

6. Un procédé selon la revendication 5, dans lequel un dérivé du N-(5-tétrazolyl)-6-phényl-2-pyridinecarboxamide de la formule

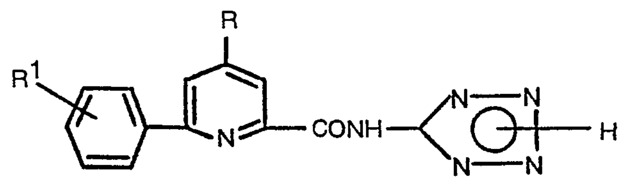

dans laquelle R est de l'hydrogène, un halogène, un alkyle (en $C_1$ à $C_4$) ou un alcoxy (en $C_1$ à $C_3$) et $R^1$ est un di-alkyle (en $C_1$ à $C_2$)-amino ou acétyl-amino, est préparé par condensation d'un dérivé de l'acide 6-phényl-2-pyridinecarboxylique de formule:

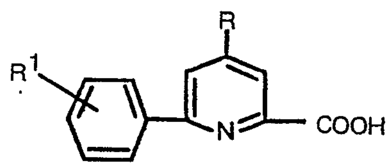

dans laquelle R et $R^1$ sont tels que définis ci-dessus, avec du 5-aminotétrazole.

7. Un procédé selon la revendication 5, dans lequel un dérivé du N-(5-tétrazolyl)-6-phényl-2-pyridinecarboxamide de la formule

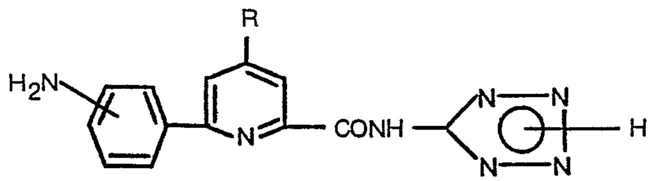

dans laquelle R est de l'hydrogène, un halogène, un alkyle (en $C_1$ à $C_4$) ou un alcoxy (en $C_1$ à $C_3$), est préparé par condensation d'un dérivé de l'acide 6-phenyl-2-pyridine-carboxylique de formule:

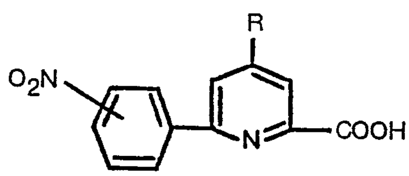

dans laquelle R est tel que défini ci-dessus, avec du 5-aminotétrazole pour obtenir un composé de formule:

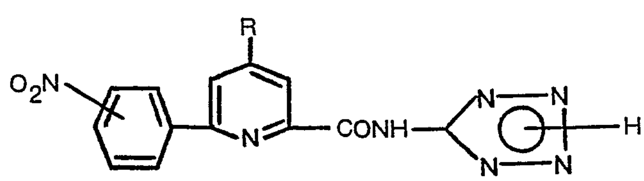

dans laquelle R est tel que défini ci-dessus, et par réduction dudit composé afin de transformer le groupe nitro en groupe amino.

8. Un procédé selon la revendication 5, dans lequel un dérivé du N-(5-tétrazolyl)-6-phényl-2-pyridinecarboxamide de formule:

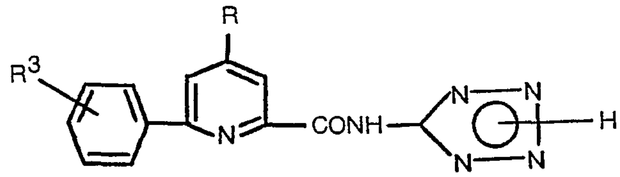

dans laquelle R est de l'hydrogène, un halogène, un alkyle (en $C_1$ à $C_4$) ou un alcoxy (en $C_1$ à $C_3$) et $R^3$ est un amino, un mono-alkyl (en $C_1$ à $C_2$)-amino, est préparé en condensant un dérivé de l'acide 6-phényl-2-pyridinecarboxylique de formule:

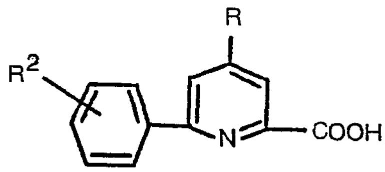

dans laquelle R est tel que défini ci-dessus et $R^2$ est un amino protégé avec un groupe amino-protecteur ou un monoalkyle (en $C_1$ à $C_2$)-amino protégé avec un groupe amino-protecteur, avec du 5-amino-tétrazole pour obtenir un composé de formule:

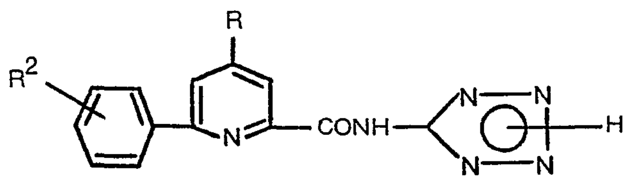

dans laquelle R et $R^2$ sont tels que définis ci-dessus, et en éliminant le groupe amino-protecteur du produit résultant.

9. Une composition pharmaceutique qui comprend un dérivé du N-(5-tétrazolyl)-6-phényl-2-pyridinecarboxamide de formule

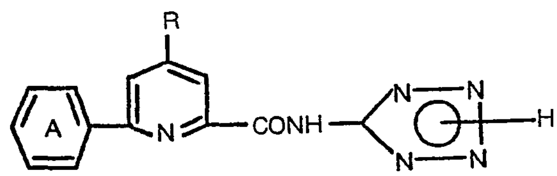

dans laquelle R est de l'hydrogène, un halogène, un alkyle (en $C_1$ à $C_4$) ou un alcoxy (en $C_1$ à $C_3$), et le noyau phénylique A est un phényle présentant un substituant choisi parmi un amino, un monoalkyle (en $C_1$ à $C_2$)-amino, un di- alkyle (en $C_1$ à $C_2$)-amino, et un acétylamino ou un de ses sels pharmaceutiquement acceptables et un support pharmaceutiquement acceptable pour lui.

**Patentansprüche**

1. N-(5-Tetrazolyl)-6-phenyl-2-pyridinecarboxamid-Derivat der Formel

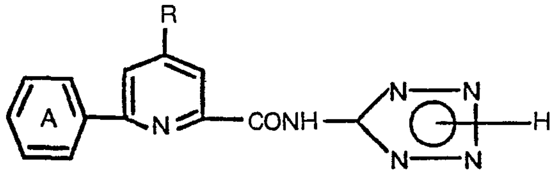

[I]

worin R Wasserstoff, ein Halogen, einen Alkyl($C_{1-4}$)- oder einen Alkoxy($C_{1-3}$)-Rest darstellt, und der Phenylring A mit einer Amino-, Mono-Alkyl($C_{1-2}$)-amino-, Dialkyl($C_{1-2}$)-amino- oder Acetylamino-Gruppe substituiert ist, oder ein pharmazeutisch annehmbares Salz desselben.

2. Verbindung gemäss Anspruch 1 dadurch gekennzeichnet, dass R Wasserstoff, Chlor, Methyl, Ethyl, Propyl, Isopropyl, Butyl, Methoxy, Ethoxy, Propoxy oder Isopropoxy darstellt.

3. Verbindung gemäss Anspruch 1 dadurch gekennzeichnet, dass der Phenylring A aus der Gruppe 3-Aminophenyl, 4-Aminophenyl, 3-Methylaminophenyl, 4-Methylaminophenyl, 3-Ethylaminophenyl, 4-Ethylaminophenyl, 3-Dimethylaminophenyl, 4-Dimethylaminophenyl, 3-Diethylaminophenyl, 4-Diethylaminophenyl, 3-Acetylaminophenyl und 4-Acetylaminophenyl ausgewählt wird.

4. Verbindung gemäss Anspruch 1, dadurch gekennzeichnet, dass diese N-(5-Tetrazolyl)-4-methyl-6-(4-methylaminophenyl)-2-pyridin-carboxamid oder ein pharmazeutisch annehmbares Salz desselben darstellt.

5. Verfahren zur Herstellung eines N-(5-Tetrazolyl)-6-phenyl-2-pyridin-carboxamid-Derivates der Formel

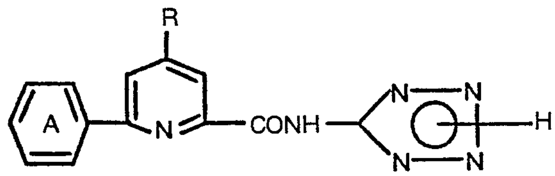

worin R Wasserstoff, ein Halogen, einen Alkyl($C_{1-4}$)- oder Alkoxy($C_{1-3}$)-Rest darstellt, und der Phenylring A mit einer Amino-, Mono-Alkyl($C_{1-2}$)-amino-, Dialkyl($C_{1-2}$)-amino- oder Acetylamino-Gruppe substituiert ist, oder ein pharmazeutisch annehmbares Salz desselben, gekennzeichnet durch kondensieren eines 6-Phenyl-2-pyridin-carbonsäure-Derivates der Formel

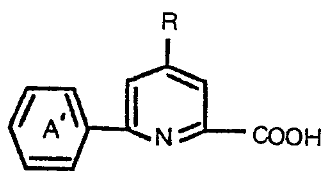

worin R die vorstehend angegebene Bedeutung hat, und der Phenylring A' einen Phenylring darstellt, welcher einen Substituenten aus der Gruppe Nitro, Dialkyl($C_{1-2}$)-amino, Acetylamino, Amino, welches mit einer Aminoschutzgruppe geschützt ist, und Mono-Alkyl($C_{1-2}$)-amino, geschützt mit einer Aminoschutzgruppe, trägt, mit 5-Aminotetrazol, wobei, wenn der Phenylring A' einen mit einer Nitrogruppe substituierten Phenylring darstellt, die genannte erhaltene Verbindung reduziert wird, um die Nitrogruppe in eine Aminogruppe umzuwandeln, und wenn der Phenylring A' ein Phenyl mit einem Substituenten aus der Gruppe Amino, geschützt durch eine Aminoschutzgruppe, und Mono-Alkyl($C_{1-2}$)-amino, geschützt mit einer Aminoschutzgruppe, darstellt, die genannte Aminoschutzgruppe von der erhaltenen Verbindung abgespalten wird.

6. Verfahren gemäss Anspruch 5, dadurch gekennzeichnet, dass ein N-(5-Tetrazolyl)-6-phenyl-2-pyridin-carboxamid-Derivat der Formel

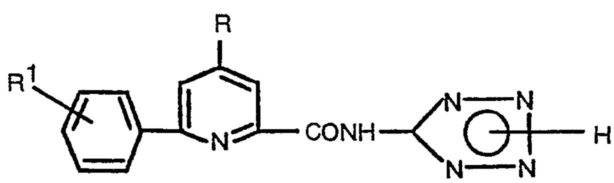

worin R Wasserstoff, ein Halogen, einen Alkyl($C_{1-4}$)- oder einen Alkoxy($C_{1-3}$)-Rest darstellt, und $R^1$ Dialkyl($C_{1-2}$)-amino oder Acetylamino bedeutet, hergestellt wird durch Kondensieren eines 6-Phenyl-2-pyridin-carbonsäure-Derivates der Formel:

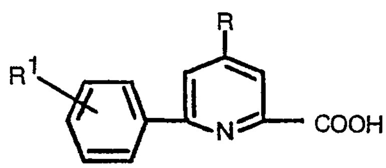

worin R und $R^1$ die vorstehend angegebenen Bedeutungen haben, mit 5-Aminotetrazol.

7. Verfahren gemäss Anspruch 5, dadurch gekennzeichnet, dass ein N-(5-Tetrazolyl)-6-phenyl-2-pyridin-carboxamid-Derivat der Formel

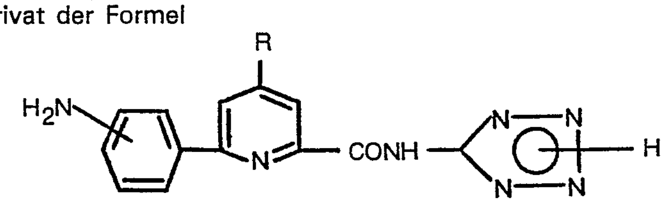

worin R Wasserstoff, ein Halogen, einen Alkyl$(C_{1-4})$- oder einen Alkoxy$(C_{1-3})$-Rest darstellt, hergestellt wird durch Kondensieren eines 6-Phenyl-2-pyridin-carbonsäure-Derivates der Formel

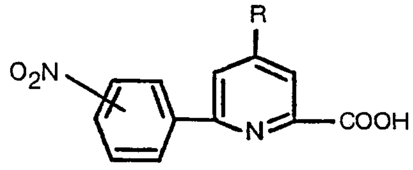

worin R die vorstehend angegebene Bedeutung hat, mit 5-Aminotetrazol unter Erhalt einer Verbindung der Formel

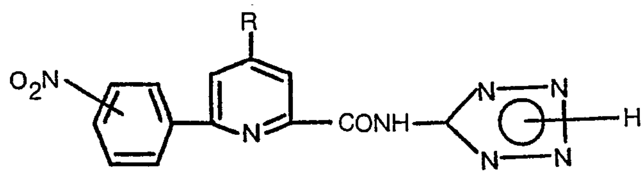

worin R die vorstehend angegebene Bedeutung hat, und Reduktion der genannten Verbindung, um die Nitrogruppe in die Aminogruppe umzuwandeln.

8. Verfahren gemäss Anspruch 5, dadurch gekennzeichnet, dass ein N-(5-Tetrazolyl)-6-phenyl-2-pyridin-carboxamid-Derivat der Formel

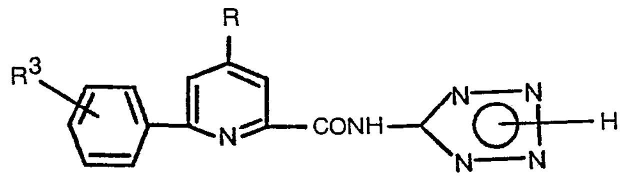

worin R Wasserstoff, ein Halogen, einen Alkyl$(C_{1-4})$- oder einen Alkoxy$(C_{1-3})$-Rest darstellt, und $R^3$ eine Aminogruppe oder eine Mono-alkyl$(C_{1-2})$-amino-Gruppe bedeutet, hergestellt wird durch Kondensieren eines 6-Phenyl-2-pyridin-carbonsäure-Derivates der Formel

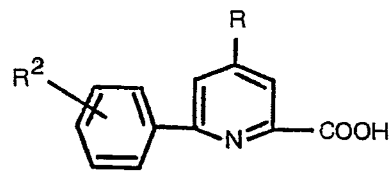

worin R die vorstehend angebene Bedeutung hat, und $R^2$ eine mit einer Aminoschutzgruppe geschützte Aminogruppe oder eine mit einer Amino-Schutzgruppe geschützte Mono-alkyl$(C_{1-2})$-amino-Gruppe darstellt, mit 5-Amino-tetrazol unter Erhalt einer Verbindung der Formel

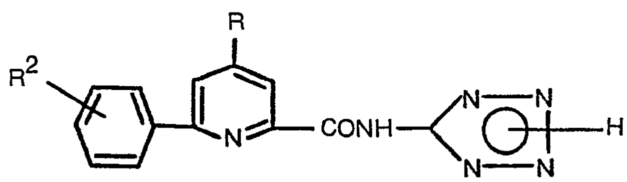

worin R und $R^2$ die vorstehend angegebenen Bedeutungen haben, und Entfernen der Aminoschutzgruppe von der erhaltenen Verbindung.

9. Pharmazeutisches Mittel, dadurch gekennzeichnet, dass es ein N-(5-Tetrazolyl)-6-phenyl-2-pyridin-carboxamid-Derivat der Formel

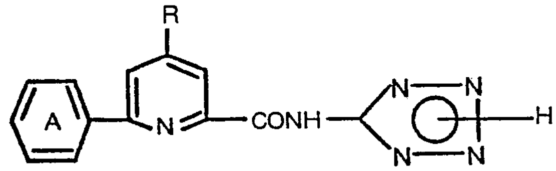

[I]

worin R Wasserstoff, ein Halogen, einen Alkyl$(C_{1-4})$- oder einen Alkoxy$(C_{1-3})$-Rest darstellt, und der Phenylring A ein Phenyl bedeutet, welcher mit einem Substituenten aus der Gruppe Amino, Mono-alkyl$(C_{1-2})$-amino, Dialkyl$(C_{1-2})$-amino und Acetylamino substituiert ist, oder ein pharmazeutisch annehmbares Salz desselben, und einen pharmazeutisch annehmbaren Träger hierzu umfasst.